# EUROPÄISCHE PATENTSCHRIFT

(11) **EP 1 325 010 B1**
(45) Veröffentlichungstag und Bekanntmachung des Hinweises auf die Patenterteilung: **27.04.2005**
(21) Anmeldenummer: 01982417.6
(22) Anmeldetag: 10.10.2001
(51) Int. Cl.: C07D 487/04, C07D 487/08, A61K 31/519, A61P 29/00

(54) **SUBSTITUIERTE 3,4-DIHYDRO-PYRIMIDO [1,2-A]PYRIMIDINE UND 3,4-DIHYDROPYRAZINO [1,2-A]PYRIMIDINE**
SUBSTITUTED 3.4-DIHYDRO-PYRIMIDO [1,2-A]PYRIMIDINES AND 3.4-DIHYDROPYRAZINO [1,2-A]PYRIMIDINES
3,4-DIHYDRO-PYRIMIDO [1,2-A]PYRIMIDINES SUBSTITUEES ET 3,4-DIHYDROPYRAZINO [1,2-A]PYRIMIDINES SUBSTITUEES

(30) Priorität: 13.10.2000 DE 10050661
(43) Veröffentlichungstag der Anmeldung: 09.07.2003
(73) Patentinhaber: Grünenthal GmbH, 52078 Aachen (DE)
(72) Erfinder: GERLACH, Matthias, 63636 Brachttal (DE); MAUL, Corinna, 52066 Aachen (DE); JAGUSCH, Utz-Peter, 52066 Aachen (DE)
(86) Internationale Anmeldenummer: PCT/EP2001/011702
(87) Internationale Veröffentlichungsnummer: WO 2002/030934

(56) Entgegenhaltungen:
- EP-A- 0 795 555
- US-A- 4 219 649

## Beschreibung

Die vorliegende Anmeldung betrifft substituierte 3,4-Dihydro-pyrimido[1,2-a]pyrimidine und 3,4-Dihydro-pyrazino[1,2-a]pyrimidine, Verfahren zu ihrer Herstellung, sie enthaltende Substanzbibliotheken, Arzneimittel, die diese Verbindungen enthalten, die Verwendung dieser Verbindungen zur Herstellung von Arzneimitteln zur Behandlung von Schmerz, Haminkontinenz, Juckreiz, Tinnitus aurium und/oder Diarrhoe sowie diese Verbindungen enthaltende pharmazeutische Zusammensetzungen.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist.

Klassische Opioide wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Der vorliegenden Erfindung liegt die Aufgabe zugrunde, analgetisch wirksame Verbindungen zur Verfügung zu stellen, die sich zur Schmerztherapie - insbesondere auch zur Therapie chronischer und neuropathischer Schmerzen - eignen. Darüber hinaus sollten diese Substanzen möglichst keine der Nebenwirkungen, die üblicherweise bei der Anwendung von Opioiden mit µ-Rezeptoraffinität wie Morphin auftreten, wie z.B. Übelkeit, Erbrechen, Abhängigkeit, Atemdepression oder Obstipation, hervorrufen.

Diese Aufgabe wird durch die Verbindungen der allgemeinen Struktur I gelöst, die analgetisch wirksam sind. Bei den erfindungsgemäßen Verbindungen handelt es sich um substituierte 3,4-Dihydro-pyrimido[1,2-a]pyrimidine und 3,4-Dihydro-pyrazino[1,2-a]pyrimidine der allgemeinen Struktur I 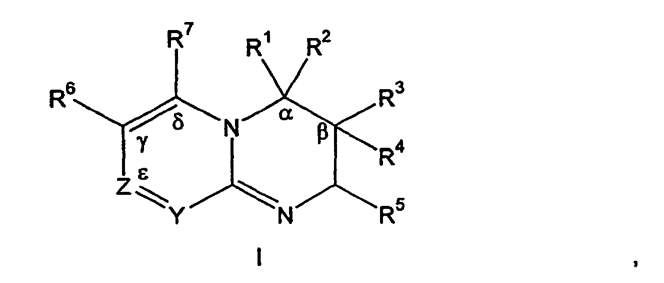 worin
- Y: CR⁸ und
- Z: N bedeutet oder
- Y: N und
- Z: CR⁹ bedeutet,
- R¹ und R²: unabhängig voneinander H, OR¹⁰, SH, SR¹⁰, C₁₋₁₂-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl 3-, 4-, 5-, 6-oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeuten, wobei einer von R¹ und R² H ist und der andere Rest von R¹ und R² nicht H ist mit der Maßgabe, daß, wenn einer von R¹ und R² Aryl bedeutet, der andere Rest von R¹ und R² H oder C₁₋₁₂-Alkyl bedeutet,
- R³ und R⁴: unabhängig voneinander H, C₁₋₁₂-Alkyl, wobei C₁₋₁₂-Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeuten, wobei mindestens einer von R³ und R⁴ H ist, oder
- einer der Reste R¹ oder R²: zusammen mit einem der Reste R³ oder R⁴ W bildet, wobei W α'-(CH₂)ₙ-β' mit n = 3, 4, 5, 6, 7, 8, 9 oder 10, α'-CH=CH-CH₂-β', α'-CH=CH-CH₂-CH₂-β', α'-CH₂-CH=CH-CH₂-β', α'-CH₂-CH₂-CH=CH-CH₂-CH₂-β', α'-O-(CH₂)ₙ-β' mit n = 2, 3, 4, 5 oder 6 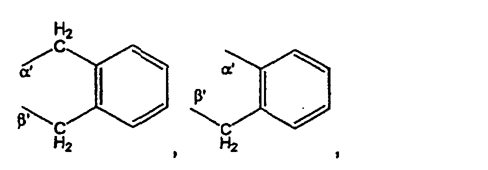 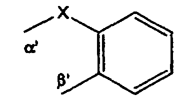 mit X = CH₂, O oder S 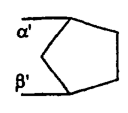 oder 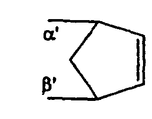 bedeutet und das mit α' gekennzeichnete Ende von W mit dem α-Kohlenstoffatom der Verbindung der allgemeinen Formel I verbunden ist und das mit β', gekennzeichnete Ende von W mit dem β-Kohlenstoffatom der Verbindung der allgemeinen Struktur I verbunden ist, der andere Rest von R¹ und R² H oder C₁₋₁₂-Alkyl bedeutet, wobei Alkyl geradkettig oder verzweigt ist und gesättigt oder ungesättigt ist und unsubstituiert oder ein- oder mehrfach substituiert ist, und der andere Rest von R³ und R⁴ H oder C₁₋₁₂-Alkyl bedeutet, wobei Alkyl geradkettig oder verzweigt ist und gesättigt oder ungesättigt ist und unsubstituiert oder einoder mehrfach substituiert ist,
- R⁵: C₁₋₁₂-Alkyl, wobei C₁₋₁₂-Alkyl geradkettig oder verzweigt ist und gesättigt oder ungesättigt ist und unsubstituiert oder einoder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyctyl 3-, 4-, 5-, 6- oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, C(=O)R¹¹, CO₂H oder CO₂R¹² bedeutet,
- R⁶, R⁷, R⁸ und R⁹: unabhängig voneinander H, F, Cl, Br, I, CN, NH₂, NH(C₁₋₆-Alkyl), N(C₁₋₆-Alkyl)₂, NH((C₁₋₆-Alkyl)-Aryl), N((C₁₋₆-Alkyl)-Aryl)₂, NH-Aryl, N(Aryl)₂, NHR₁₃, NO₂, OH, SH, O-C₁₋₈-Alkyloder S(O)ₚ-C₁₋₈-Alkyl, wobei Alkyl geradkettig oder verzweigt ist und gesättigt oder ungesättigt ist und unsubstituiert oder ein- oder mehrfach substituiert ist und p 0, 1 oder 2 ist, O-Aryl oder S(O)_{q}-Aryl, wobei Aryl unsubstituiert oder ein- oder mehrfach substituiert ist und q 0, 1 oder 2 ist, O-(C₁₋₆-Alkyl)-Aryl oder S(O)ᵣ-(C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist und r 0, 1 oder 2 ist, CO₂H, C(=O)R¹⁴, C₁₋₁₂-Alkyl, wobei Alkyl geradkettig oder verzweigt ist und gesättigt oder ungesättigt ist und unsubstituiert oder ein- oder mehrfach substituiert ist, CF₃, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl 3-, 4-, 5-, 6-oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, oder Heteroaryl, wobei Heteroaryl unsubstituiert oder einoder mehrfach substituiert ist, bedeuten oder R⁶ und R⁷ zusammen Q bilden, wobei Q γ'-CR¹⁵=CR¹⁶-CR¹⁷=CR¹⁸-δ' bedeutet, das mit γ' gekennzeichnete Ende von Q mit dem γ-Kohlenstoffatom der Verbindung der allgemeinen Struktur I verbunden ist und das mit δ' gekennzeichnete Ende von Q mit dem δ-Kohlenstoffatom der Verbindung der allgemeinen Struktur I verbunden ist, und Y und Z wie oben definiert sind, oder R⁶ und R⁹ zusammen T bilden, wobei T γ'-CR¹⁹=CR²⁰-CR²¹=CR²²-ε' oder γ'-N=CR²⁰-CR²¹=N-ε' bedeutet, das mit γ' gekennzeichnete Ende von T mit dem γ-Kohlenstoffatom der Verbindung der allgemeinen Struktur I und das mit ε' gekennzeichnete Ende von T mit dem ε-Kohlenstoffatom der Verbindung der allgemeinen Struktur I verbunden ist, und R⁷ und R⁸ wie oben definiert sind,
- R¹⁰: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl 3-, 4-, 5-, 6- oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeutet,
- R¹¹: NH₂, NH(C₁₋₆-Alkyl), N(C₁₋₆-Alkyl)₂, NH((C₁₋₆-Alkyl)-Aryl), N((C₁₋₆-Alkyl)-Aryl)₂, NH-Aryl, N(Aryl)₂, C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl,
wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeutet,
- R¹²: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeutet,
- R¹³: C(=O)CH₃, C(=O)Phenyl, C(=O)O-t.-Butyl (t-BOC) oder eine andere übliche Amino-Schutzgruppe bedeutet,
- R¹⁴: H, NH₂, NH(C₁₋₆-Alkyl), N(C₁₋₆-Alkyl)₂, NH((C₁₋₆-Alkyl)-Aryl), N((C₁₋₆-Alkyl)-Aryl)₂, NH-Aryl, N(Aryl)₂, C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, oder (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeutet, OC₁₋₈-Alkyl, wobei Alkyl geradkettig oder verzweigt ist und gesättigt oder ungesättigt ist und unsubstituiert oder ein- oder mehrfach substituiert ist, OC₃₋₈-Cycloalkyl, wobei Cycloalkyl gesättigt oder ungesättigt ist und unsubstituiert oder ein- oder mehrfach substituiert ist, OAryl, wobei Aryl unsubstituiert oder ein- oder mehrfach substituiert ist, oder O-(C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeutet,
- R¹⁵, R¹⁶, R¹⁷ und R¹⁸: unabhängig voneinander H, F, Cl, Br, I, OH, CN, C₁₋₈-Alkyl, wobei Alkyl geradkettig oder verzweigt ist und gesättigt oder ungesättigt ist und unsubstituiert oder ein- oder mehrfach substituiert ist, CO₂H bedeuten, und
- R¹⁹, R²⁰, R²¹ und R²²: unabhängig voneinander H, F, Cl, Br, I, CN, OH, C₁₋₈-Alkyl, wobei Alkyl geradkettig oder verzweigt ist und gesättigt oder ungesättigt ist und unsubstituiert oder ein- oder mehrfach substituiert ist, oder CO₂H bedeuten,
sowie ihre pharmazeutisch annehmbaren Salze.

Die Ausdrücke "C₁₋₆-Alkyl", "C₁₋₈-Alkyl" und "C₁₋₁₂-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 6 bzw. 1 bis 8 bzw. 1 bis 12 Kohlenstoffatomen, d.h. C₁₋₆-Alkanyle, C₂₋₆-Alkenyle und C₂₋₆-Alkinyle bzw. C₁₋₈-Alkanyle, C₂₋₈-Alkenyle und C₂₋₈-Alkinyle bzw. C₁₋₁₂-Alkanyle, C₂₋₁₂-Alkenyle und C₂₋₁₂-Alkinyle . Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl, n-Octyl, n-Decyl, n-Dodecyl; Ethylenyl (Vinyl), Ethinyl, Propenyl (-CH₂CH=CH₂, -CH=CH-CH₃, -C(=CH₂)-CH₃), Propinyl (-CH-C≡CH), Butenyl, Butinyl, Pentenyl, Pentinyl, Hexenyl, Hexinyl, Octenyl und Octinyl umfaßt.

Der Ausdruck "C₃₋₈-Cycloalkyl" bedeutet für die Zwecke dieser Erfindung cyclische Kohlenwasserstoffe mit 3 bis 8 Kohlenstoffatomen, die gesättigt oder ungesättigt, unsubstituiert oder ein- oder mehrfach substituiert sein können. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält. Für die Zwecke der vorliegenden Erfindung besonders bevorzugt sind Cyclopropyl, Cyclopropyl-2-carbonsäure, Cyclopropyl-2-carbonsäureethylester und Cyclohexyl.

Der Ausdruck "Aryl" bedeutet im Sinne dieser Erfindung aromatische Kohlenwasserstoffe, u.a. Phenyle, Naphthyle und Anthracenyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen Position des Aryls sein können. Vorteilhafterweise ist Aryl aus der Gruppe ausgewählt, die Phenyl, 1-Naphthyl und 2-Naphthyl enthält. Besonders bevorzugte Aryl-Reste sind für die Zwecke dieser Erfindung m-Toluyl, p-Hydroxy-phenyl, p-Methoxyphenyl, 4-Hydroxy-3-methoxyphenyl, 3,4-Dimethoxyphenyl, 2,4-Dimethylphenyl, 4-Fluorphenyl, 1-Naphthyl und 2-Naphthyl.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Heteroarylsubstituenten gleich oder verschieden sein und in jeder beliebigen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der HeteroarylRest ausgewählt ist aus der Gruppe, die Pyrrolyl, Indolyl, Furyl (Furanyl), Benzofuranyl, Thienyl (Thiophenyl), Benzothienyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, Isochinolinyl, Chinazolinyl, Carbazolyl, Phenazinyl, Phenothiazinyl enthält, wobei die Bindung an die Verbindungen der allgemeinen Struktur I über jedes beliebige und mögliche Ringglied des Heteroaryl-Restes erfolgen kann. Besonders bevorzugte Heteroaryl-Reste sind für die Zwecke dieser Erfindung Pyridin-2-yl, Furan-2-yl, 5-Methyl-furan-2-yl und 5-Nitro-furan-2-yl.

Die Ausdrücke "(C₁₋₆-Alkyl)-Aryl" bzw. "(C₁₋₆-Alkyl)-Heteroaryl" bedeuten für die Zwecke der vorliegenden Erfindung, daß C₁₋₆-Alkyl, Aryl und Heteroaryl die oben definierten Bedeutungen haben und über eine C₁₋₆-Alkyl-Gruppe an die Verbindung der allgemeinen Struktur I gebunden sind.

Der Ausdruck "Heterocyclyl" steht für einen 3-, 4-, 5-, 6- oder 7-gliedrigen cyclischen organischen Rest, der mindestens 1, ggf. auch 2, 3, 4 oder 5 Heteroatome enthält, wobei die Heteroatome gleich oder verschieden sind und der cyclische Rest gesättigt oder ungesättigt, aber nicht aromatisch ist, und unsubstituiert oder ein- oder mehrfach substituiert sein kann. Der Heterocyclus kann auch Teil eines bi- oder polycyclischen Systems sein. Bevorzugte Heteroatome sind Stickstoff, Sauerstoff und Schwefel. Es ist bevorzugt, daß der Heterocyclyl-Rest ausgewählt ist aus der Gruppe, die Tetrahydrofuryl, Tetrahydropyranyl, Pyrrolidinyl, Piperidinyl, Piperazinyl und Morpholinyl enthält, wobei die Bindung an die Verbindung der allgemeinen Struktur I (bzw. II, III oder IV) über jedes beliebige Ringglied des Heterocyctyl-Restes erfolgen kann.

Im Zusammenhang mit "Alkyl", "Alkanyl", "Alkenyl" und "Alkinyl" versteht man unter dem Begriff "substituiert" im Sinne dieser Erfindung die Substitution eines Wasserstoffrestes durch F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Besonders bevorzugt für die Zwecke der vorliegenden Erfindung bedeutet Alkyl Methyl, Ethyl, CH₂-CH₂OH oder CF₃.

In Bezug auf "Aryl", "Alkyl-Aryl", "Heteroaryl", "Alkyl-Heteroaryl", "Heterocyclyl", sowie "Cycloalkyl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Cycloalkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, S(O)-Alkyl, S(O)-Aryl, SO₂-Alkyl, SO₂-Aryl, SO₂NH₂, SO₃H, Cycloalkyl, Aryl, Heteroaryl, CF₃, =O, =S; C₁₋₆-Alkanyl, C₂-₆-Alkenyl, C₂₋₆-Alkinyl, -C₁-₆-Alkyl-C(O)O-C₁-₆-Alkyl; Phenyl, Benzyl, Naphthyl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" sind dabei besonders bevorzugte Substituenten OH, F, CH₃ und O-CH₃. Für "Heteroaryl" sind besonders bevorzugte Substituenten CH₃ und NO₂. Für "Cycloalkyl" sind besonders bevorzugte Substituenten CO₂H und CO₂Ethyl. Pharmazeutisch annehmbare Salze im Sinne dieser Erfindung sind solche Salze der erfindungsgemäßen Verbindungen gemäß der allgemeinen Struktur I, die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden.

Vorzugsweise werden die pharmazeutisdsch annehmbaren Salze der erfindungsgemäßen Verbindungen gemäß der allgemeinen Struktur I mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Ebenfalls bevorzugt sind die Hydrate der erfindungsgemäßen Verbindungen, die z.B. durch Kristallisation aus wäßriger Lösung erhalten werden können.

Alle erfindungsgemäßen Verbindungen enthalten mindestens ein Asymmetriezentrum, nämlich das mit R⁵ substituierte Kohlenstoffatom der Struktur I. Daher können die erfindungsgemäßen Verbindungen der allgemeinen Struktur I in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen. Bevorzugt liegen die Verbindungen der allgemeinen Struktur I als enantiomerenreine Verbindungen vor.

Eine Gruppe bevorzugter Verbindungen der vorliegenden Erfindung wird von 3,4-Dihydro-pyrimido[1,2-a]pyrimidinen, d.h. Verbindungen der allgemeinen Struktur I mit Y = N und Z = CR⁹, gebildet, in denen einer der Reste R¹ und R² OR¹⁰, SR¹⁰, C₁₋₆-Alkyl oder Aryl bedeutet, einer der Reste R³ und R⁴ H oder C₁₋₆-Alkyl bedeutet, oder einer der Reste R¹ und R² zusammen mit einem der Reste R³ und R⁴ W bildet, wobei W α'-CH=CH-CH₂-CH₂-β', 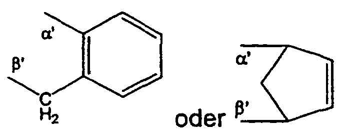 bedeutet, und die beiden anderen Reste von R¹, R², R³ und R⁴ unabhängig voneinander H oder C₁₋₁₂-Alkyl bedeuten, R⁵ C₃₋₇-Cycloalkyl, Heteroaryl, C(=O)R¹¹, CO₂H oder CO₂R¹² bedeutet, R⁶ H, F, Cl, Br, CN, NO₂, C(=O)R¹⁴, C₁₋₆-Alkyl, CF₃ oder Aryl bedeutet, R⁷ H, F, Cl, Br, CN, NH₂, OH oder C₁₋₆-Alkyl bedeutet, R⁹ H, OH, CF₃ oder C₁₋₆-Alkyl bedeutet oder R⁶ und R⁹ gemeinsam T bilden, wobei T γ'-N=CR²⁰-CR²¹=N-ε' bedeutet, R¹⁰ C₁₋₈-Alkyl oder Aryl bedeutet, R¹¹ Aryl bedeutet, R¹² C₁₋₆-Alkyl bedeutet, R¹⁴ OC₁₋₆-Alkyl bedeutet, R²⁰ H oder CO₂H bedeutet und R²¹ H bedeutet.

Besonders bevorzugte Verbindungen dieser Gruppe sind hierbei 3,4-Dihydro-pyrimido[1,2-a]pyrimidine der allgemeinen Struktur IA 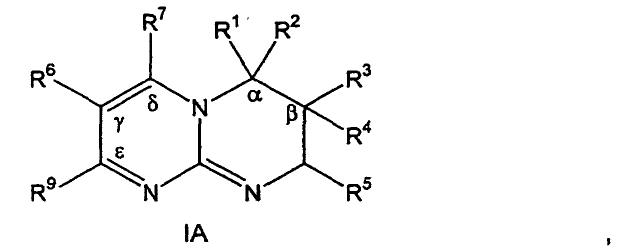 in denen einer der Reste R¹ und R² O-(CH₂)₂-OH, S-Phenyl, Phenyl, 3-Methylphenyl, 2,4-Dimethylphenyl, 4-Hydroxyphenyl, 4-Methoxyphenyl, 4-Hydroxy-3-methoxyphenyl, 3,4-Dimethoxyphenyl oder 2-Naphthyl bedeutet, einer der Reste R³ und R⁴ H oder Methyl bedeutet, oder einer der Reste R¹ und R² zusammen mit einem der Reste R³ und R⁴ W bildet, wobei W α'-CH=CH-CH₂-CH₂-β', 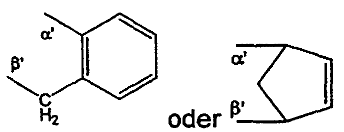 bedeutet, und die beiden anderen Reste von R¹, R², R³ und R⁴ H bedeuten, R⁵ Cyclopropyl, 2-(C(=O)O-Ethyl)-cyclopropyl, Cyclohexyl, 2-Pyridinyl, C(=O)Phenyl, CO₂H oder CO₂Ethyl, R⁶ H, Br, CO₂Ethyl oder Methyl bedeutet, R⁷ H, NH₂, OH oder Methyl bedeutet und R⁹ H, CI, OH oder CF₃ bedeutet oder R⁸ und R⁹ gemeinsam T bilden, wobei T γ'-N=C(CO₂H)-CH=N-ε' bedeutet.

Die am meisten bevorzugten erfindungsgemäßen Dihydro-pyrimido[1,2-a]pyrimidine der allgemeinen Struktur IA sind jene, die ausgewählt sind aus:
7-Brom-4-(4-methoxy-phenyl)-3,4-dihydro-2H-pyrimido[1,2-a]pyrimidin-2-carbonsäureethylester,
7-Brom-4-(3,4-dimethoxy-phenyl)-3,4-dihydro-2H-pyrimido[1,2-a]pyrimidin-2-carbonsäure,
7-Brom-4-(4-methoxy-phenyl)-3,4-dihydro-2H-pyrimido[1,2-a]pyrimidin-2-carbonsäure,
7-Brom-4-(4-hydroxy-phenyl)-3-methyl-3,4-dihydro-2H-pyrimido[1,2-a]pyrimidin-2-carbonsäureethylester,
2-Brom-6,6a,7,11b-tetrahydro-4,5,11c-triaza-benzo[c]fluoren-6-carbonsäureethylester,
7-Brom-4-(4-methoxy-phenyl)-3-methyl-3,4-dihydro-2H-pyrimido[12-a]pyrimidin-2-carbonsäure,
7-Brom-4-(3,4-dimethoxy-phenyl)-3,4-dihydro-2H-pyrimido[1,2-a]pyrimidin-2-carbonsäure,
7-Brom-4-(4-methoxy-phenyl)-3,4-dihydro-2H-pyrimido[1,2-a]pyrimidin-2-carbonsäure,
[7-Brom-4-(2-hydroxy-ethoxy)-3,4-dihydro-2H-pyrimido[1,2-a]pyrimidin-2-yl]-phenyl-methanon,
[7-Brom-4-(4-hydroxy-3-methoxy-phenyl)-3-methyl-3,4-dihydro-2Hpyrimido[1,2-a]pyrimidin-2-yl]-phenyl-methanon,
[7-Brom-4-(4-methoxy-phenyl)-3-methyl-3,4-dihydro-2H-pyrimido[1,2-a]pyrimidin-2-yl]-phenyl-methanon,
[7-Brom-4-(4-hydroxy-phenyl)-3-methyl-3,4-dihydro-2H-pyrimido[1,2-a]pyrimidin-2-yl]-phenyl-methanon,
[7-Brom-4-(3,4-dimethoxy-phenyl)-3,4-dihydro-2H-pyrimido[1,2-a]pyrimidin-2-yl]-phenyl-methanon,
[7-Brom-4-(4-methoxy-phenyl)-3,4-dihydro-2H-pyrimido[1,2-a]pyrimidin-2-yl]-phenyl-methanon,
2-(7-Brom-2-cyclopropyl-3,4-dihydro-2H-pydmido[1,2-a]pyrimidin-4-yloxy)-ethanol,
2-(7-Brom-2-cyclohexyl-3,4-dihydro-2H-pyrimido[1,2-a]pyrimidin-4-yloxy)-ethanol,
4-(7-Brom-2-cyclohexyl-3-methyl-3,4-dihydro-2H-pyrimido[1,2-a]pyrimidin-4-yl)-phenol,
7-Brom-4-naphthalin-2-yl-3,4-dihydro-2H-pyrimido[1,2-a]pyrimidin-2-carbonsäureethylester, .
7-Brom-4-m-tolyl-3,4-dihydro-2H-pyrimido[1,2-a]pyrimidin-2-carbonsäureethylester,
2-[7-Brom-4-(2,4-dimethyl-phenyl)-3,4-dihydro-2H-pyrimido[1,2-a]pyrimidin-2-yl]-cyclopropancarbonsäureethylester,
2-[7-Brom-4-(4-methoxy-phenyl)-3,4-dihydro-2H-pyrimido[1,2-a]pyrimidin-2-yl]-cyclopropancarbonsäureethylester,
7-Brom-4-phenylsulfanyl-2-pyridin-2-yl-3,4-dihydro-2H-pyrimido[1,2-a]pyrimidin,
2-Chlor-6-phenylsulfanyl-8-pyridin-2-yl-7,8-dihydro-6H-pyrimido[1,2-a]pyrimidin-4-yl-amin,
6-Phenylsulfanyl-8-pyridin-2-yl-7,8-dihydro-6H-pyrimido[1,2-a]pyrimidin-2,4-diol,
6-Phenylsulfanyl-8-pyridin-2-yl-2-trifluormethyl-7,8-dihydro-6H-pyrimido[1,2-a]pyrimidin-3-carbonsäureethylester,
3-Methyl-5,8-methano-9-pyridin-2-yl-5,8,8a,9-tetrahydro-4bH-1,4a,10-triaza-phenanthren-2,4-diol,
5,8-Methano-9-pyridin-2-yl-5,8,8a,9-tetrahydro-4bH-1,4a,10-triazaphenanthren-2,4-diol und
12-Hydroxyl-1,4-methano-5-pyridin-2-yl-1,4a,5,12b-tetrahydro-4H-6,7,8,11,12a-pentaaza-benzo[a]anthracen-10-carbonsäure,
3-Bromo-9-pyridin-2-yl-7,8,8a,9-tetrahydro-4bH-1,4a,10-triazaphenanthren,
sowie ihre pharmazeutisch annehmbaren Salze.

Eine weitere Gruppe bevorzugter Verbindungen wird von 3,4-Dihydropyrazino[1,2-a]pyrimidinen, d.h. Verbindungen der allgemeinen Struktur I mit Y = CR⁸ und Z = N, gebildet, in denen einer der Reste R¹ und R² H, OR¹⁰, SR¹⁰, C₁₋₆-Alkyl oder Aryl bedeutet, einer der Reste R³ und R⁴ H oder C₁₋₆-Alkyl bedeutet, oder einer der Reste R¹ und R² zusammen mit einem der Reste R³ und R⁴ W bildet, wobei W 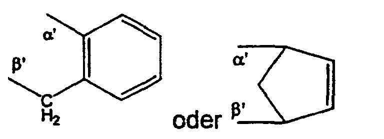 bedeutet, und die beiden anderen Reste von R¹, R², R³ und R⁴ H oder C₁₋₁₂-Alkyl bedeuten, R⁵ C₃₋₇-Cycloalkyl, Heteroaryl, C(=O)R¹¹, CO₂H oder CO₂R¹² bedeutet, R⁶ H, F, Cl, Br, CN, NO₂, C(=O)R¹⁴, C₁₋₆-Alkyl, CF₃ oder Aryl bedeutet, R⁷ H, F, Cl, Br, CN, NH₂, OH oder C₁₋₆-Alkyl bedeutet, R⁸ H, F, Cl, Br, CN, NO₂, O-(C₁₋₆-Alkyl)-Aryl, CO₂H, CONH₂ oder C₁₋₆-Alkyl bedeutet, R¹⁰ C₁₋₈-Alkyl oder Aryl bedeutet, R¹¹ Aryl bedeutet, R¹² C₁₋₆-Alkyl bedeutet und R¹⁴ OC₁₋₆-Alkyl bedeutet. Diese Verbindungen können auch in Form eines ihrer pharmazeutisch annehmbaren Salze vorliegen.

Besonders bevorzugte Verbindungen dieser Gruppe sind hierbei 3,4-Dihydro-pyrazino[1,2-a]pyrimidine der allgemeinen Struktur IB 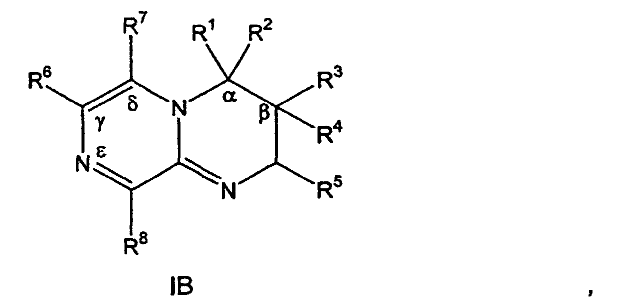 in denen einer der Reste R¹ und R² O-(CH₂)₂-OH, S-Phenyl, Methyl, Phenyl, 2,4-Dimethyl-phenyl, 4-Hydroxyphenyl, 4-Methoxyphenyl, 4-Hydroxy-3-methoxy-phenyl oder 3,4-Dimethoxy-phenyl bedeutet, einer der Reste R³ und R⁴ H oder Methyl bedeutet, oder einer der Reste R¹ und R² zusammen mit einem der Reste R³ und R⁴ W bildet, wobei W 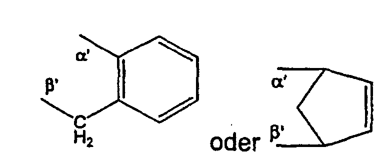 bedeutet, und die beiden anderen Reste von R¹, R², R³ und R⁴ H bedeuten, R⁵ Cyclopropyl, 2-(C(=O)O-Ethyl)-cyclopropyl, Cyclohexyl, 2-Pyridinyl, 5-Methyl-furan-2-yl, 5-Nitro-furan-2-yl, C(=O)Phenyl, CO₂H oder CO₂Ethyl, R⁶ H, Cl, CN oder Phenyl bedeutet, R⁷ H, NH₂ oder CN bedeutet und R⁸ H, Cl, CN, CO₂H oder CONH₂ bedeutet, sowie ihre pharmazeutisch annehmbaren Salze.

Die am meisten bevorzugten erfindungsgemäßen Dihydro-pyrazino[1,2-a]pyrimidine der allgemeinen Struktur IB sind jene, die ausgewählt sind aus:
4-(4-Methoxy-phenyl)-3-methyl-2-(5-nitro-furan-2-yl)-3,4-dihydro-2Hpyrazino[1,2-a]pyrimidin,
2-[4-(4-Methoxy-phenyl)-3-methyl-3,4-dihydro-2H-pyrazino[1,2-a]pyrimidin-2-yl]-cyclopropancarbonsäureethylester,
4-(4-Methoxy-phenyl)-3-methyl-2-(5-methyl-furan-2-yl)-3,4-dihydro-2Hpyrazino[1,2-a]pyrimidin,
4-(3,4-Dimethoxy-phenyl)-3,4-dihydro-2H-pyrazino[1,2-a]pyrimidin-2-carbonsäureethylester,
4-(4-Methoxy-phenyl)-3,4-dihydro-2H-pyrazino[1,2-a]pyrimidin-2-carbonsäure ethylester,
4-(3,4-Dimethoxy-phenyl)-3,4-dihydro-2H-pyrazino[1,2-a]pyrimidin-2-carbonsäure,
4-(4-Methoxy-phenyl)-3,4-dihydro-2H-pyrazino[1,2-a]pydrnidin-2-carbonsäure,
4-(4-Methoxy-phenyl)-2-(5-nitro-furan-2-yl)-3,4-dihydro-2H-pyrazino[1,2-a]pyrimidin,
2-[4-(2,4-Dimethyl-phenyl)-3,4-dihydro-2H-pyrazino[1,2-a]pyrimidin-2-yl]-cyclopropancarbonsäureethylester,
2-[4-(4-Methoxy-phenyl)-3,4-dihydro-2H-pyrazino[1,2-a]pyrimidin-2-yl]-cyclopropancarbonsäureethylester,
4-(4-Hydroxy-phenyl)-3-methyl-3,4-dihydro-2H-pyrazino[1,2-a]pyrimidin-2-carbonsäureethylester,
6,6a,7,11b-Tetrahydro-3,5,11c-triaza-benzo[c]fluorene-6-carbonsäureethylester,
4-Phenylsulfanyl-3,4-dihydro-2H-pyrazino[1,2-a]pyrimidin-2-carbonsäureethylester,
4-Phenylsulfanyl-3,4-dihydro-2H-pyrazino[1,2-a]pyrimidin-2-carbonsäure,
4-(4-Methoxy-phenyl)-3-methyl-3,4-dihydro-2H-pyrazino[1,2-a]pyrimidin-2-carbonsäure,
4-(3,4-Dimethoxy-phenyl)-3,4-dihydro-2H-pyrazino[1,2-a]pyrimidin-2-carbonsäure,
4-(4-Methoxy-phenyl)-3,4-dihydro-2H-pyrazino[1,2-a]pyrimidin-2-carbonsäure,
[4-(2-Hydroxy-ethoxy)-3,4-dihydro-2H-pyrazino[1,2-a]pyrimidin-2-yl]-phenylmethanon,
[4-(4-Hydroxy-3-methoxy-phenyl)-3-methyl-3,4-dihydro-2H-pyrazino[1,2-a]pyrimidin-2-yl]-phenyl-methanon,
[4-(4-Methoxy-phenyl)-3-methyl-3,4-dihydro-2H-pyrazino[1,2-a]pyrimidin-2-yl]-phenyl-methanon,
[4-(4-Hydroxy-phenyl)-3-methyl-3,4-dihydro-2H-pyrazino[1,2-a]pyrimidin-2-yl]-phenyl-methanon,
2-Cyclopropyl-4-(3,4-dimethoxy-phenyl)-3,4-dihydro-2H-pyrazino[1,2-a]pyrimidin,
2-Cyclopropyl-4-(4-methoxy-phenyl)-3,4-dihydro-2H-pyrazino[1,2-a]pyrimidin,
2-(2-Cyclohexyl-3,4-dihydro-2H-pyrazino[1,2-a]pyrimidin-4-yloxy)-ethanol,
2-Cyclohexyl-4-(4-methoxy-phenyl)-3,4-dihydro-2H-pyrazino[1,2-a]pyrimidin,
4-Methyl-2-(5-nitro-furan-2-yl)-4-phenyl-3,4-dihydro-2H-pyrazino[1,2-a]pyrimidin,
2-(4-Methyl-4-phenyl-3,4-dihydro-2H-pyrazino[1,2-a]pyrimidin-2-yl)-cyclopropancarbonsäureethylester,
4-Phenyl-2-pyridin-2-yl-3,4-dihydro-2H-pyrazino[1,2-a]pyrimidin,
4-Phenyl-2-pyridin-2-yl-3,4-dihydro-2H-pyrazino[1,2-a]pyrimidin-9-carbonsäure,
6-Amino-7-chlor-4-phenyl-2-pyridin-2-yl-3,4-dihydro-2H-pyrazino[1,2-a]pyrimidin-9-carbonsäureamid,
2-Phenylsulfanyl-3-pyridin-2-yl-2,3-dihydro-1H-pyrimido[1,2-a]chinolin-10-ol,
4-Phenylsulfanyl-2-pyridin-2-yl-3,4-dihydro-2H-pyrazino[1,2-a]pyrimidin-9-carbonsäure,
6-Amino-7-chlor-4-phenylsulfanyl-2-pyridin-2-yl-3,4-dihydro-2Hpyrazino[1,2-a]pyrimidin-9-carbonsäureamid,
7-Phenyl-4-phenylsulfanyl-2-pyridin-2-yl-3,4-dihydro-2H-pyrazino[1,2-a]pyrimidin-9-carbonitril,
5,8-Methano-9-pyridin-2-yl-5,8,8a,9-tetrahydro-4bH-2,4a,10-triazaphenanthren,
1-Chlor-5,8-methano-9-pyridin-2-yl-5,8,8a,9-tetrahydro-4bH-2,4a,10-triazaphenanthren-3,4-dicarbonitril,
sowie ihre pharmazeutisch annehmbaren Salze.

Die Erfindung betrifft auch ein Verfahren zur Herstellung der erfindungsgemäßen Verbindungen der Struktur I und ihrer pharmazeutisch annehmbaren Salze 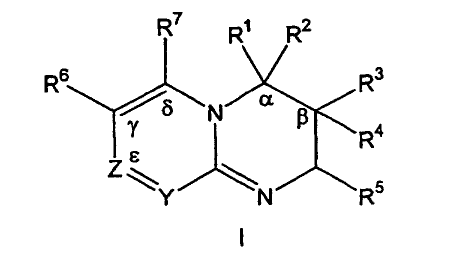 worin
Y, Z und R¹ bis R⁷ wie oben definiert sind,
das dadurch gekennzeichnet ist, daß
ein Heteroarylamin der allgemeinen Struktur II 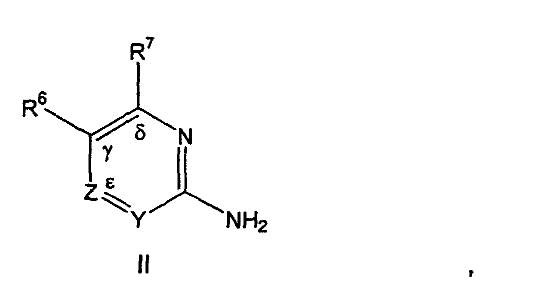 worin Y, Z, R⁶ und R⁷ wie oben definiert sind, mit der Maßgabe, daß, wenn R⁶ und R⁷ das wie oben definierte Q bilden, das mit γ' gekennzeichnete Ende von Q mit dem γ-Kohlenstoffatom des Heteroarylamins der allgemeinen Struktur II und das mit δ' gekennzeichnete Ende von Q mit dem δ-Kohlenstoffatom des Heteroarylamins der allgemeinen Struktur II verbunden ist, und daß, wenn R⁶ und R⁹ das wie oben definierte T bilden, das mit γ' gekennzeichnete Ende mit dem γ-Kohlenstoffatom des Heteroarylamins der allgemeinen Struktur II und das mit ε' gekennzeichnete Ende mit dem ε-Kohlenstoffatom des Heteroarylamins der allgemeinen Struktur II verbunden ist,
mit einem Aldehyd der allgemeinen Struktur III 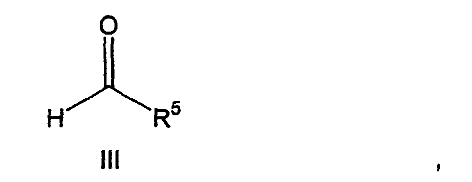 worin R⁵ wie oben definiert ist,
und einem Olefin der allgemeinen Struktur IV 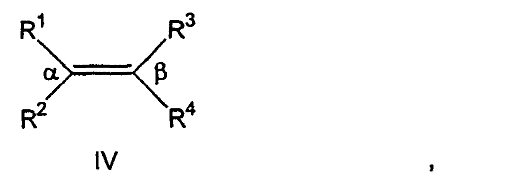 worin R¹, R², R³ und R⁴ wie oben definiert sind, mit der Maßgabe, daß wenn einer der Reste R¹ und R² zusammen mit einem der Reste R³ und R⁴ W bildet, das mit α' gekennzeichnete Ende von W mit dem α-Kohlenstoffatom des Olefins der allgemeinen Struktur IV und das mit β' gekennzeichnete Ende von W mit dem β-Kohlenstoffatom des Olefins der allgemeinen Struktur IV verbunden ist, in Gegenwart einer Säure umgesetzt wird.

Zur Herstellung der erfindungsgemäßen 3,4-Dihydro-pyrimido[1,2-a]pyrimidine der allgemeinen Struktur IA werden in dem erfindungsgemäßen Verfahren Heteroarylamine der allgemeinen Struktur IIA 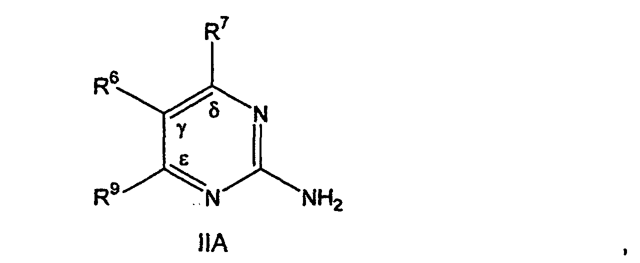 worin R⁶, R⁷ und R⁹ wie oben für Formel II definiert sind, eingesetzt. Zur Herstellung der erfindungsgemäßen 3,4-Dihydro-pyrazino[1,2-a]pyrimidine der allgemeinen Struktur IB werden in dem erfindungsgemäßen Verfahren Heteroarylamine der allgemeinen Struktur IIB 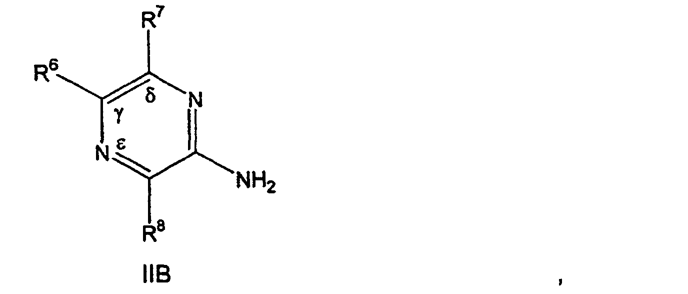 worin R⁶ bis R⁸ wie oben für Formel II definiert sind, eingesetzt.

Das erfindungsgemäße Verfahren wird bevorzugt in einer "Eintopf"-Reaktion durchgeführt, bei der je ein Heteroarylamin der allgemeinen Struktur 11, je ein Aldehyd der allgemeinen Struktur III und je ein Olefin der allgemeinen Struktur IV gleichzeitig miteinander umgesetzt werden.

Das erfindungsgemäße Verfahren kann auch in semi- oder vollautomatisierter Form als Parallelsynthese einer Gruppe von erfindungsgemäßen Verbindungen der allgemeinen Struktur I durchgeführt werden.

Bei der eingesetzten Säure handelt es sich um eine anorganische oder organische Protonen- oder Lewis-Säure. Bevorzugt wird die Reaktion in Gegenwart einer organischen Säure, z.B. Essigsäure, Methansulfonsäure oder insbesondere Trifluoressigsäure, durchgeführt.

Das erfindungsgemäße Herstellungsverfahren kann in jedem geeigneten Lösungsmittel durchgeführt werden, in dem die Reaktanten sich ausreichend lösen. Bevorzugt sind als Lösungsmittel organische Solventien, z.B. Dichlormethan oder insbesondere Acetonitril.

Die erfindungsgemäße Herstellung der erfindungsgemäßen Verbindungen der allgemeinen Struktur I erfolgt zweckmäßig bei einer Temperatur von 0 bis 100 °C, insbesondere bei 15 bis 40°C. Die Reaktionszeit beträgt vorzugsweise 15 Minuten bis 12 Stunden und kann den jeweiligen Erfordernissen angepaßt werden.

Die im erfindungsgemäßen Verfahren eingesetzten Heteroarylamine der allgemeinen Struktur II, die Aldehyde der allgemeinen Struktur III und die Olefine der allgemeinen Struktur IV sind käuflich erhältlich (von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCl, Japan) oder können nach im Stand der Technik allgemein bekannten Verfahren hergestellt werden.

Die erfindungsgemäßen Verbindungen der allgemeinen Struktur I können sowohl als freie Base als auch als Salz isoliert werden. Die freie Base der Verbindung der allgemeinen Struktur I wird üblicherweise nach erfolgter Umsetzung gemäß dem oben dargelegten erfindungsgemäßen Verfahren und anschließender herkömmlicher Aufarbeitung erhalten. Die so gewonnene Base kann dann beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Die besonders bevorzugte Hydrochloridbildung kann auch durch Versetzen der in einem geeigneten organischen Lösungsmittel gelösten Base mit Trimethylsilylchlorid (TMSCI) herbeigeführt werden.

Soweit die Verbindungen der allgemeinen Struktur I in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, gebildeten diastereomeren Salzen voneinander getrennt werden.

Ein weiterer Gegenstand der vorliegenden Erfindung ist eine Substanzbibliothek, die mindestens eine Verbindung der wie oben definierten allgemeinen Struktur I enthält. Vorzugsweise enthält die erfindungsgemäße Substanzbibliothek mindestens 15 und insbesondere mindestens 30 Verbindungen der allgemeinen Struktur I.

Dabei wird für die Zwecke der vorliegenden Erfindung unter einer "Substanzbibliothek" eine Gruppe von Verbindungen verstanden, die nach dem gleichen Verfahren unter gleichen oder nahezu gleichen Reaktionsbedingungen und unter Variation eines Reagenzes oder mehrerer Reagenzien hergestellt werden. Eine solche Substanzbibliothek kann die Bibliotheksmitglieder sowohl als einzelne reine Verbindungen als auch als Mischung dieser Verbindungen enthalten. Mit Hilfe dieser Substanzbibliothek kann beispielsweise ein medizinisches Screening in einem oder mehreren in-vitro-Screening-Verfahren in automatisierter Form durchgeführt werden.

Die erfindungsgemäßen Verbindungen haben sich als analgetisch wirksam erwiesen. Daher ist auch ein Arzneimittel, das mindestens eine der erfindungsgemäßen und wie oben definierten Verbindungen der allgemeinen Struktur I bzw. eines ihrer pharmazeutisch annehmbaren Salze umfaßt, Gegenstand der vorliegenden Erfindung. Dabei können die erfindungsgemäßen Verbindungen in dem erfindungsgemäßen Arzneimittel als isomerenreine, insbesondere enantiomerenreine bzw. diastereomerenreine, Verbindungen, aber auch als racemisches oder nicht-racemisches Gemisch vorliegen. Bevorzugt ist dabei, daß das Arzneimittel ein pharmazeutisch annehmbares Salz der erfindungsgemäßen Verbindungen enthält, insbesondere ein Hydrochlorid.

Darüber hinaus ist ein weiterer Gegenstand der Erfindung die Verwendung mindestens einer erfindungsgemäßen Verbindung der allgemeinen Struktur I, einschließlich ihrer Diastereomeren oder Enantiomeren, auch als Racemate oder Enantiomerengemisch in Form ihrer freien Base oder eines mit einer physiologisch verträglichen Säure gebildeten Salzes, insbesondere des Hydrochloridsalzes, zur Herstellung eines Arzneimittels zur Behandlung von Schmerz.

Überraschenderweise hat es sich herausgestellt, daß die erfindungsgemäßen Verbindungen der allgemeinen Struktur I für weitere Indikationen, insbesondere zur Behandlung von Haminkontinenz, Juckreiz, Tinnitus aurium und/oder Diarrhoe, sehr geeignet sind. Ein weiterer Gegenstand der Erfindung ist daher die Verwendung mindestens einer erfindungsgemäßen Verbindung der allgemeinen Struktur I einschließlich eines pharmazeutisch annehmbaren Salzes zur Herstellung eines Arzneimittels zur Behandlung von Harninkontinenz, Juckreiz, Tinnitus aurium und/oder Diarrhoe.

Darüber hinaus sind auch pharmazeutische Zusammensetzungen Gegenstand der vorliegenden Erfindung, die mindestens eine Verbindung der wie oben definierten allgemeinen Struktur I oder eines ihrer pharmazeutisch annehmbaren Salze und einen oder mehrere pharmazeutische Hilfsstoffe enthalten.

Die erfindungsgemäßen Arzneimittel und pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, transdermale therapeutische Systeme, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Struktur I je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, vaginal, pulmonal, intraperitoneal, transdermal, intramuskulär, nasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Pulver zur Inhalation sowie Sprays. Erfindungsgemäße Verbindungen der allgemeinen Struktur I in einem Depot in gelöster Form oder in einem Pflaster, gegebenenfalls unter Zusatz von die Hautpenetration fördernden Mitteln, sind geeignete perkutane Applikationszubereitungen. Rektal, transmucosal, parenteral, oral oder perkutan anwendbare Zubereitungsformen können die erfindungsgemäßen Verbindungen der allgemeinen Struktur I verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Struktur I appliziert.

Die nachfolgenden Beispiele dienen zur näheren Erläuterung der vorliegenden Erfindung.

### Beispiele

Die eingesetzten Chemikalien und Lösungsmittel wurden kommerziell von einem der folgenden Anbieter erworben: Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan; oder nach allgemeinen im Stand der Technik bekannten Verfahren hergestellt.

Die chromatographische Reinigung erfolgte an einer HPLC-RP-18-Säule der Fa. Macherey-Nagel; Material NUCLEOSIL 100-3 C₁₈-HD. ca. 100 mm (VarioPrep), Innendurchmesser 21 mm; Laufmittel Wasser/Methanol, Gradient: 50-100 % in ca. 18 min., Fluß: 10 ml/min.; Detektion: UV, Beckman 168 PDA.

### Allgemeine Arbeitsvorschrift AAV (Semiautomatisierte Synthese)

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde in den auf 20 °C temperierten Rührblock gestellt. Anschließend wurden nacheinander die folgenden Reagenzien hinzupipettiert:
1 ml einer Lösung aus Trifluoressigsäure, 0,1 M, und
Heteroarylaminkomponente II, 0,1 M, in Acetonitril;
1 ml einer 0,11 M Lösung des Aldehyds III in Acetonitril;
1 ml einer 0,3 M Lösung des Olefins IV in Acetonitril.

Das Reaktionsgemisch wurde bei 20 °C in einem der Rührblöcke 10 h lang gerührt. Danach wurde die Reaktionslösung abfiltriert. Das Röhrchen wurde dabei zweimal mit je 1,5 ml einer 7,5% NaHCO₃-Lösung gespült.

Das Rack mit den Proben wurde manuell auf die Aufarbeitungsanlage gestellt. Das Reaktionsgemisch wurde auf einem Vortexer mit 2 ml Ethylacetat versetzt und geschüttelt. Zur Ausbildung der Phasengrenze wurde in der Zentrifuge kurz zentrifugiert. Die Phasengrenze wurde optisch detektiert und die organische Phase abpipettiert. Im nächsten Schritt wurde die wäßrige Phase erneut mit 2 ml Ethylacetat versetzt, geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt.

Jede Probe wurde mit ESI-MS und/oder NMR charakterisiert. Massenspektrometrische Untersuchungen (ESI-MS) wurden mit einem Massenspektrometer der Fa. Finnegan, LCQ Classic durchgeführt. ¹H-NMR-Untersuchungen der erfindungsgemäßen Verbindungen wurden mit einem 300 MHz DPX Advance NMR-Gerät der Fa. Bruker durchgeführt.

Nach der angegebenen AAV wurden die Beispiele 1 bis 61 (s. Tabelle 1) hergestellt. Die Beispiele 43 bis 45 wurden mittels reversed-phase-HPLC aufgereinigt.

**Tabelle 1**

| **Beispiel** | **berechnete Masse** | **gefundene Masse** | **Name** |
|---|---|---|---|
| 1 | 366,37 | 367,3 | 4-(4-Methoxy-phenyl)-3-methyl-2-(5-nitrofuran-2-yl)-3,4-dihydro-2H-pyrazino[1,2-a]pyrimidin |
| 2 | 367,44 | 368,4 | 2-[4-(4-Methoxy-phenyl)-3-methyl-3,4-dihydro-2H-pyrazino[1,2-a]pyrimidin-2-yl]-cyclopropancarbonsäureethylester |
| 3 | 335,4 | 336,2 | 4-(4-Methoxy-phenyl)-3-methyl-2-(5-methylfuran-2-yl)-3,4-dihydro-2H-pyrazino[1,2-a]pyrimidin |
| 4 | 343,38 | 344,2 | 4-(3,4-Dimethoxy-phenyl)-3,4-dihydro-2H-pyrazino[1,2-a]pyrimidin-2-carbonsäureethylester |
| 5 | 313,35 | 314,1 | 4-(4-Methoxy-phenyl)-3,4-dihydro-2H-pyrazino[1,2-a]pyrimidin-2-carbonsäure ethylester |
| 6 | 392,25 | 391,1/393,1 | 7-Brom-4-(4-methoxy-phenyl)-3,4-dihydro-2H-pyrimido[1,2-a]pyrimidin-2-carbonsäureethylester |
| 7 | 315,32 | 316,2 | 4-(3,4-Dimethoxy-phenyl)-3,4-dihydro-2H-pyrazino[1,2-a]pyrimidin-2-carbonsäure |
| 8 | 394,22 | 393,2/395,1 | 7-Brom-4-(3,4-dimethoxy-phenyl)-3,4-dihydro-2H-pyrimido[1,2-a]pyrimidin-2-carbonsäure |
| 9 | 285,3 | 286,2 | 4-(4-Methoxy-phenyl)-3,4-dihydro-2H-pyrazino[1,2-a]pyrimidin-2-carbonsäure |
| 10 | 364,2 | 363,2/365,0 | 7-Brom-4-(4-methoxy-phenyl)-3,4-dihydro-2H-pyrimido[1,2-a]pyrimidin-2-carbonsäure |
| 11 | 352,34 | 353,4 | 4-(4-Methoxy-phenyl)-2-(5-nitro-furan-2-yl)-3,4-dihydro-2H-pyrazino[1,2-a]pyrimidin |
| 12 | 351,44 | 352,4 | 2-[4-(2,4-Dimethyl-phenyl)-3,4-dihydro-2H-pyrazino[1,2-a]pyrimidin-2-yl]-cyclopropancarbonsäureethylester |
| 13 | 353,42 | 354,3 | 2-[4-(4-Methoxy-phenyl)-3,4-dihydro-2H-pyrazino[1,2-a]pyrimidin-2-yl]-cyclopropancarbonsäureethylester |
| 14 | 313,35 | 314,3 | 4-(4-Hydroxy-phenyl)-3-methyl-3,4-dihydro-2H-pyrazino[1,2-a]pyrimidin-2-carbonsäureethylester |
| 15 | 392,25 | 391,4/393,0 | 7-Brom-4-(4-hydroxy-phenyl)-3-methyl-3,4-dihydro-2H-pyrimido[1,2-a]pyrimidin-2-carbonsäureethylester |
| 16 | 295,34 | 296,3 | 6,6a,7,11b-Tetrahydro-3,5,11c-triazabenzo[c]fluorene-6-carbonsäureethylester |
| 17 | 374,24 | 373,3/375,3 | 2-Brom-6,6a,7,11b-tetrahydro-4,5,11c-triazabenzo[c]fluoren-6-carbonsäureethylester |
| 18 | 315,39 | 316,3 | 4-Phenylsulfanyl-3,4-dihydro-2H-pyrazino[1,2-a]pyrimidin-2-carbonsäureethylester |
| 19 | 287,34 | 288,2 | 4-Phenylsulfanyl-3,4-dihydro-2H-pyrazino[1,2-a]pyrimidin-2-carbonsäure |
| 20 | 299,32 | 300,2 | 4-(4-Methoxy-phenyl)-3-methyl-3,4-dihydro-2H-pyrazino[1,2-a]pyrimidin-2-carbonsäure |
| 21 | 378,23 | 377,1/379,1 | 7-Brom-4-(4-methoxy-phenyl)-3-methyl-34-dihydro-2H-pyrimido[12-a]pyrimidin-2-carbonsäure |
| 22 | 315,32 | 316,2 | 4-(3,4-Dimethoxy-phenyl)-3,4-dihydro-2H-pyrazino[1,2-a]pyrimidin-2-carbonsäure |
| 23 | 394,22 | 393,1/395,1 | 7-Brom-4-(3,4-dimethoxy-phenyl)-3,4-dihydro-2H-pyrimido[1,2-a]pyrimidin-2-carbonsäure |
| 24 | 285,3 | 286,2 | 4-(4-Methoxy-phenyl)-3,4-dihydro-2H-pyrazino[1,2-a]pyrimidin-2-carbonsäure |
| 25 | 364,2 | 363,1/365,1 | 7-Brom-4-(4-methoxy-phenyl)-3,4-dihydro-2H-pyrimido[1,2-a]pyrimidin-2-carbonsäure |
| 26 | 299,32 | 300,2 | [4-(2-Hydroxy-ethoxy)-3,4-dihydro-2H-pyrazino[1,2-a]pyrimidin-2-yl]-phenyl-methanon |
| 27 | 378,23 | 379,1 | [7-Brom-4-(2-hydroxy-ethoxy)-3,4-dihydro-2H-pyrimido[1,2-a]pyrimidin-2-yl]-phenyl-methanon |
| 28 | 375,42 | 374,2/376,2 | [4-(4-Hydroxy-3-methoxy-phenyl)-3-methyl-3,4-dihydro-2H-pyrazino[1,2-a]pyrimidin-2-yl]-phenyl-methanon |
| 29 | 454,32 | 451,2/453,1 | [7-Brom-4-(4-hydroxy-3-methoxy-phenyl)-3-methyl-3,4-dihydro-2H-pyrimido[1,2-a]pyrimidin-2-yl]-phenyl-methanon |
| 30 | 359,42 | 360,2 | [4-(4-Methoxy-phenyl)-3-methyl-3,4-dihydro-2H-pyrazino[1,2-a]pyrimidin-2-yl]-phenyl-methanon |
| 31 | 438,32 | 437,1/439,1 | [7-Brom-4-(4-methoxy-phenyl)-3-methyl-3,4-dihydro-2H-pyrimido[1,2-a]pyrimidin-2-yl]-phenyl-methanon |
| 32 | 345,4 | 346,2 | [4-(4-Hydroxy-phenyl)-3-methyl-3,4-dihydro-2H-pyrazino[1,2-a]pyrimidin-2-yl]-phenyl-methanon |
| 33 | 424,3 | 423,1/425,1 | [7-Brom-4-(4-hydroxy-phenyl)-3-methyl-3,4-dihydro-2H-pyrimido[1,2-a]pyrimidin-2-yl]-phenyl-methanon |
| 34 | 454,32 | 453,2/455,1 | [7-Brom-4-(3,4-dimethoxy-phenyl)-3,4-dihydro-2H-pyrimido[1,2-a]pyrimidin-2-yl]-phenyl-methanon |
| 35 | 424,3 | 423,2/425,1 | [7-Brom-4-(4-methoxy-phenyl)-3,4-dihydro-2H-pyrimido[1,2-a]pyrimidin-2-yl]-phenyl-methanon |
| 36 | 314,18 | 313,1/315,1 | 2-(7-Brom-2-cyclopropyl-3,4-dihydro-2H-pyrimido[1,2-a]pyrimidin-4-yloxy)-ethanol |
| 37 | 311,38 | 312,2 | 2-Cyclopropyl-4-(3,4-dimethoxy-phenyl)-3,4-dihydro-2H-pyrazino[1,2-a]pyrimidin |
| 38 | 281,35 | 282,2 | 2-Cyclopropyl-4-(4-methoxy-phenyl)-3,4-dihydro-2H-pyrazino[1,2-a]pyrimidin |
| 39 | 277,36 | 278,2 | 2-(2-Cyclohexyl-3,4-dihydro-2H-pyrazino[1,2-a]pyrimidin-4-yloxy)-ethanol |
| 40 | 356,26 | 357,2 | 2-(7-Brom-2-cyclohexyl-3,4-dihydro-2H-pyrimido[1,2-a]pyrimidin-4-yloxy)-ethanol |
| 41 | 402,34 | 403,1 | 4-(7-Brom-2-cyclohexyl-3-methyl-3,4-dihydro-2H-pyrimido[1,2-a]pyrimidin-4-yl)-phenol |
| 42 | 323,43 | 324,2 | 2-Cyclohexyl-4-(4-methoxy-phenyl)-3,4-dihydro-2H-pyrazino[1,2-a]pyrimidin |
| 43 | 412,29 | 413,1 | 7-Brom-4-naphthalin-2-yl-3,4-dihydro-2H-pyrimido[1,2-a]pyrimidin-2-carbonsäureethylester |
| 44 | 376,25 | 375,4/377,4 | 7-Brom-4-m-tolyl-3,4-dihydro-2H-pyrimido[1,2-a]pyrimidin-2-carbonsäureethylester |
| 45 | 430,35 | 431,1 | 2-[7-Brom-4-(2,4-dimethyl-phenyl)-3,4-dihydro-2H-pyrimido[1,2-a]pyrimidin-2-yl]-cyclopropancarbonsäureethylester |
| 46 | 432,32 | 431,4/433,4 | 2-[7-Brom-4-(4-methoxy-phenyl)-3,4-dihydro-2H-pyrimido[1,2-a]pyrimidin-2-yl]-cyclopropancarbonsäureethylester |
| 47 | 336,35 | 337,3 | 4-Methyl-2-(5-nitro-furan-2-yl)-4-phenyl-3,4-dihydro-2H-pyrazino[1,2-a]pyrimidin |
| 48 | 339,44 | 338,3 | 2-(4-Methyl-4-phenyl-3,4-dihydro-2H-pyrazino[1,2-a]pyrimidin-2-yl)-cyclopropancarbonsäureethylester |
| 49 | 320,41 | 321,2 | 4-Phenylsulfanyl-2-pyridin-2-yl-3,4-dihydro-2H-pyrazino[1,2-a]pyrimidin |
| 50 | 364,42 | 365,2 | 4-Phenylsulfanyl-2-pyridin-2-yl-3,4-dihydro-2H-pyrazino[1,2-a]pyrimidin-9-carbonsäure |
| 51 | 412,90 | 413,3 | 6-Amino-7-chlor-4-phenylsulfanyl-2-pyridin-2-yl-3,4-dihydro-2H-pyrazino[1,2-a]pyrimidin-9-carbonsäureamid |
| 52 | 421,52 | 422,4 | 7-Phenyl-4-phenylsulfanyl-2-pyridin-2-yl-3,4-dihydro-2H-pyrazino[1,2-a]pyrimidin-9-carbonitril |
| 53 | 343,23 | 343,2 | 7-Brom-4-phenylsulfanyl-2-pyridin-2-yl-3,4-dihydro-2H-pyrimido[1,2-a]pyrimidin |
| 54 | 369,87 | 370,3 | 2-Chlor-6-phenylsulfanyl-8-pyridin-2-yl-7,8-dihydro-6H-pyrimido[1,2-a]pyrimidin-4-yl-amin |
| 55 | 352,41 | 353,3 | 6-Phenylsulfanyl-8-pyridin-2-yl-7,8-dihydro-6H-pyrimido[1,2-a]pyrimidin-2,4-diol |
| 56 | 460,48 | 461,3 | 6-Phenylsulfanyl-8-pyridin-2-yl-2-trifluormethyl-7,8-dihydro-6H-pyrimido[1,2-a]pyrimidin-3-carbonsäureethylester |
| 57 | 276,35 | 277,4 | 5,8-Methano-9-pyridin-2-yl-5,8,8a,9-tetrahydro-4bH-2,4a,10-triaza-phenanthren |
| 58 | 360,82 | 324,3 (M - Cl) | 1-Chlor-5,8-methano-9-pyridin-2-yl-5,8,8a,9-tetrahydro-4bH-2,4a,10-triaza-phenanthren-3,4-dicarbonitril |
| 59 | 388,40 | 324,1 (M - COOH - OH) | 12-Hydroxy-1,4-methano-5-pyridin-2-yl-1,4a,5,12b-tetrahydro-4H-6,7,8,11,12a-pentaaza-benzo[a]anthracen-10-carbonsäure |
| 60 | 322,36 | 323,2 | 3-Methyl-5,8-methano-9-pyridin-2-yl-5,8,8a,9-tetrahydro-4bH-1,4a,10-triaza-phenanthren-2,4-diol |
| 61 | 308,35 | 309,3 | 5,8-Methano-9-pyridin-2-yl-5,8,8a,9-tetrahydro-4bH-1,4a,10-hiaza-phenanthren-2,4-diol |
| 62 | 343,23 | 343,2 | 3-Bromo-9-pyridin-2-yl-7,8,8a,9-tetrahydro-4bH-1,4a,10-triaza-phenanthren |

### Pharmakologische Tests

Erfindungsgemäße Verbindungen wurden nach von J. P. Devlin in "High throughput screening - the discovery of bioactive substances", Marcel Dekker, New York, 1997, S. 275-453, beschriebenen Verfahren auf ihre pharmakologischen Eigenschaften untersucht. Ergebnisse dieser Tests sind in den Tabellen 2 und 3 zusammengefaßt und belegen die analgetische Wirksamkeit der erfindungsgemäßen Verbindungen.

**Tabelle 2:**

| Kᵢ-Wert der µ-Opiat-Rezeptor-Bindung | |
|---|---|
| Beispiel | Kᵢ[µM] |
| 51 | 1,4 |
| 52 | 1,4 |
| 53 | 2,5 |

**Tabelle 3:**

| % Hemmung der NMDA/MK801-Bindungsstelle | |
|---|---|
| Beispiel | % Hemmung [10 µM] |
| 51 | 40 |
| 52 | 47 |
| 53 | 44 |
| 61 | 40 |
| 62 | 40 |

### Pharmazeutische Formulierung eines erfindungsgemäßen Arzneimittels

1 g des Hydrochlorids von 4-(3,4-Dimethoxy-phenyl)-3,4-dihydro-2Hpyrazino[1,2-a]pyrimidin-2-carbonsäure wurde in 1 I Wasser für Injektionszwecke bei Raumtemperatur gelöst und anschließend durch Zugabe von Natriumchlorid auf isotone Bedingungen eingestellt.

## Patentansprüche

1. Verbindung der allgemeinen Struktur I 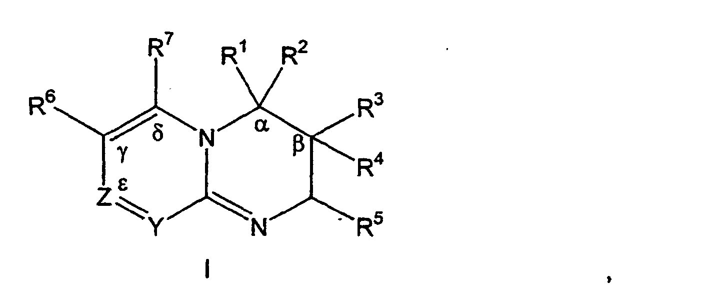 worin
Y CR⁸ und
Z N bedeutet oder
Y N und
Z CR⁹ bedeutet,
R¹ und R² unabhängig voneinander H, OR¹⁰, SH, SR¹⁰, C₁₋₁₂-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl 3-, 4-, 5-, 6- oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeuten, wobei einer von R¹ und R² H ist und der andere Rest von R¹ und R² nicht H ist mit der Maßgabe, daß, wenn einer von R¹ und R² Aryl bedeutet, der andere Rest von R¹ und R² H oder C₁₋₁₂-Alkyl bedeutet,
R³ und R⁴ unabhängig voneinander H, C₁₋₁₂-Alkyl, wobei C₁₋₁₂-Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeuten, wobei mindestens einer von R³ und R⁴ H ist, oder
einer der Reste R¹ oder R² zusammen mit einem der Reste R³ oder R⁴ W bildet, wobei W α'-(CH₂)ₙ-β' mit n = 3, 4, 5, 6, 7, 8, 9 oder 10, α'-CH=CH-CH₂-β', α'-CH=CH-CH₂-CH₂-β', α'-CH₂-CH=CH-CH₂-β', α'-CH₂-CH₂-CH=CH-CH₂-CH₂-β', α'-O-(CH₂)ₙ-β' mit n = 2, 3, 4, 5 oder 6, 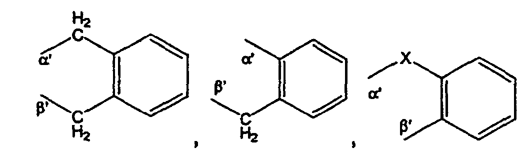 mit X = CH₂, O oder S, 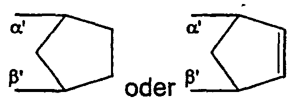 bedeutet und das mit α' gekennzeichnete Ende von W mit dem α-Kohlenstoffatom der Verbindung der allgemeinen Formel I verbunden ist und das mit β' gekennzeichnete Ende von W mit dem β-Kohlenstoffatom der Verbindung der allgemeinen Struktur I verbunden ist, der andere Rest von R¹ und R² H oder C₁₋₁₂-Alkyl ist, wobei Alkyl geradkettig oder verzweigt ist und gesättigt oder ungesättigt ist und unsubstituiert oder ein- oder mehrfach substituiert ist, und der andere Rest von R³ und R⁴ H oder C₁₋₁₂-Alkyl ist, wobei Alkyl geradkettig oder verzweigt ist und gesättigt oder ungesättigt ist und unsubstituiert oder ein- oder mehrfach substituiert ist,
R⁵ C₁₋₁₂-Alkyl, wobei C₁₋₁₂-Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl 3-, 4-, 5-, 6- oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, C(=O)R¹¹, CO₂H oder CO₂R¹² bedeutet,
R⁶, R⁷, R⁸ und R⁹ unabhängig voneinander H, F, Cl, Br, I, CN, NH₂, NH(C₁₋₆-Alkyl), N(C₁₋₆-Alkyl)₂, NH((C₁₋₆-Alkyl)-Aryl), N((C₁₋₆-Alkyl)-Aryl)₂, NH-Aryl, N(Aryl)₂, NHR¹³, NO₂, OH, SH, O-C₁₋₈-Alkyl oder S(O)ₚ-C₁₋₈-Alkyl, wobei Alkyl geradkettig oder verzweigt ist und gesättigt oder ungesättigt ist und unsubstituiert oder ein- oder mehrfach substituiert ist und p 0, 1 oder 2 ist, O-Aryl oder S(O)_{q}-Aryl, wobei Aryl unsubstituiert oder ein- oder mehrfach substituiert ist und q 0, 1 oder 2 ist, O-(C₁₋₆-Alkyl)-Aryl oder S(O)ᵣ-(C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder einoder mehrfach substituiert ist und r 0, 1 oder 2 ist, CO₂H, C(=O)R¹⁴, C₁₋₁₂-Alkyl, wobei Alkyl geradkettig oder verzweigt ist und gesättigt oder ungesättigt ist und unsubstituiert oder ein- oder mehrfach substituiert ist, CF₃, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl 3-, 4-, 5-, 6- oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, oder Heteroaryl, wobei Heteroaryl unsubstituiert oder ein- oder mehrfach substituiert ist, bedeuten oder R⁶ und R⁷ zusammen Q bilden, wobei Q γ'-CR¹⁵=CR¹⁶-CR¹⁷=CR¹⁸-δ' bedeutet, das mit γ' gekennzeichnete Ende von Q mit dem γ-Kohlenstoffatom der Verbindung der allgemeinen Struktur I verbunden ist und das mit δ' gekennzeichnete Ende von Q mit dem δ-Kohlenstoffatom der Verbindung der allgemeinen Struktur I verbunden ist, und Y und Z wie oben definiert sind, oder R⁶ und R⁹ zusammen T bilden, wobei T γ'-CR¹⁹=CR²⁰-CR²¹=CR²²-ε' oder γ'-N=CR²⁰-CR²¹=N-ε' bedeutet, das mit γ' gekennzeichnete Ende von T mit dem γ-Kohlenstoffatom der Verbindung der allgemeinen Struktur I und das mit ε' gekennzeichnete Ende von T mit dem ε-Kohlenstoffatom der Verbindung der allgemeinen Struktur I verbunden ist, und R⁷ und R⁸ wie oben definiert sind,
R¹⁰ C₁₋₈-Alkyl, wobei Alkyl geradkettig oder verzweigt ist und gesättigt oder ungesättigt ist und unsubstituiert oder einoder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl 3-, 4-, 5-, 6- oder 7-gliedrig ist und gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeutet,
R¹¹ NH₂, NH(C₁₋₆-Alkyl), N(C₁₋₆-Alkyl)₂, NH((C₁₋₆-Alkyl)-Aryl), N((C₁₋₆-Alkyl)-Aryl)₂, NH-Aryl, N(Aryl)₂, C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, oder (C₁₋₆-Alkyl)-Heteroaryl, wobei C₁₋₆-Alkyl geradkettig oder verzweigt ist und gesättigt oder ungesättigt ist und unsubstituiert oder ein- oder mehrfach substituiert ist und Heteroaryl unsubstituiert oder ein- oder mehrfach substituiert ist, bedeutet,
R¹² C₁₋₈-Alkyl, wobei Alkyl geradkettig oder verzweigt ist und gesättigt oder ungesättigt ist und unsubstituiert oder einoder mehrfach substituiert ist, C₃₋₈-Cycloalkyl, wobei Cycloalkyl gesättigt oder ungesättigt ist und unsubstituiert oder ein- oder mehrfach substituiert ist, (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeutet,
R¹³ C(=O)CH₃, C(=O)Phenyl oder C(=O)O-t.-Butyl (t-BOC) bedeutet,
R¹⁴ H, NH₂, NH(C₁₋₆-Alkyl), N(C₁₋₆-Alkyl)₂, NH((C₁₋₆-Alkyl)-Aryl), N((C₁₋₆-Alkyl)-Aryl)₂, NH-Aryl, N(Aryl)₂, C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, oder (C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder einoder mehrfach substituiert ist, bedeutet, OC₁₋₈-Alkyl, wobei Alkyl geradkettig oder verzweigt ist und gesättigt oder ungesättigt ist und unsubstituiert oder ein- oder mehrfach substituiert ist, OC₃₋₈-Cycloalkyl, wobei Cycloalkyl gesättigt oder ungesättigt ist und unsubstituiert oder ein- oder mehrfach substituiert ist, OAryl, wobei Aryl unsubstituiert oder ein- oder mehrfach substituiert ist, oder O-(C₁₋₆-Alkyl)-Aryl, wobei C₁₋₆-Alkyl geradkettig oder verzweigt ist und gesättigt oder ungesättigt ist und unsubstituiert oder ein- oder mehrfach substituiert ist und Aryl unsubstituiert oder einoder mehrfach substituiert ist, bedeutet,
R¹⁵, R¹⁶, R¹⁷ und R¹⁸ unabhängig voneinander H, F, Cl, Br, I, OH, CN, C₁₋₈-Alkyl, wobei Alkyl geradkettig oder verzweigt ist und gesättigt oder ungesättigt ist und unsubstituiert oder ein- oder mehrfach substituiert ist, CO₂H bedeuten, und
R¹⁹, R²⁰, R²¹ und R²² unabhängig voneinander H, F, Cl, Br, I, CN, OH, C₁₋₈-Alkyl, wobei Alkyl geradkettig oder verzweigt ist und gesättigt oder ungesättigt ist und unsubstituiert oder ein- oder mehrfach substituiert ist, oder CO₂H bedeuten,
sowie ihre pharmazeutisch annehmbaren Salze.

2. Verbindungen der allgemeinen Struktur I nach Anspruch 1 in Form ihrer Racemate, in Form der reinen Enantiomeren oder in Form von Mischungen der Enantiomeren oder Diastereomeren in einem beliebigen Mischungsverhältnis, sowie ihre pharmazeutisch annehmbaren Salze.

3. Verbindungen der allgemeinen Struktur I nach einem der Ansprüche 1 und 2 oder ihre pharmazeutisch annehmbaren Salze, wobei Y N und Z CR⁹ bedeuten, einer der Reste R¹ und R² OR¹⁰, SR¹⁰, C₁₋₆-Alkyl oder Aryl bedeutet, einer der Reste R³ und R⁴ H oder C₁₋₆-Alkyl bedeutet, oder einer der Reste R¹ und R² zusammen mit einem der Reste R³ und R⁴ W bildet, wobei W α'-CH=CH-CH₂-CH₂-β', 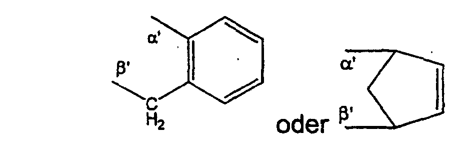 bedeutet, und die beiden anderen Reste von R¹, R², R³ und R⁴ H oder C₁₋₁₂-Alkyl bedeuten, R⁵ C₃₋₇-Cycloalkyl, Heteroaryl, C(=O)R¹¹, CO₂H oder CO₂R¹² bedeutet, R⁶ H, F, Cl, Br, CN, NO₂, C(=O)R¹⁴, C₁₋₆-Alkyl, CF₃ oder Aryl bedeutet, R⁷ H, F, Cl, Br, CN, NH₂, OH oder C₁₋₆-Alkyl bedeutet, R⁹ H, OH, CF₃ oder C₁₋₆-Alkyl bedeutet oder R⁶ und R⁹ gemeinsam T bilden, wobei T γ'-N=CR²⁰-CR²¹=N-ε' bedeutet, R¹⁰ C₁₋₈-Alkyl oder Aryl bedeutet, R¹¹ Aryl bedeutet, R¹² C₁₋₆-Alkyl bedeutet, R¹⁴ OC₁₋₆-Alkyl bedeutet, R²⁰ H oder CO₂H bedeutet und R²¹ H bedeutet.

4. Verbindungen der allgemeinen Struktur I nach Anspruch 3 oder ihre pharmazeutisch annehmbaren Salze, wobei einer der Reste R¹ und R² O-(CH₂)₂-OH, S-Phenyl, Phenyl, 3-Methylphenyl, 2,4-Dimethylphenyl, 4-Hydroxyphenyl, 4-Methoxyphenyl, 4-Hydroxy-3-methoxyphenyl, 3,4-Dimethoxyphenyl oder 2-Naphthyl bedeutet, einer der Reste R³ und R⁴ H oder Methyl bedeutet, oder einer der Reste R¹ und R² zusammen mit einem der Reste R³ und R⁴ W bildet, wobei W α'-CH=CH-CH₂-CH₂-β', 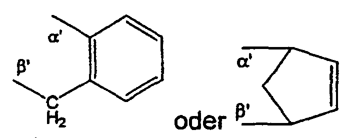 bedeutet, und die beiden anderen Reste von R¹, R², R³ und R⁴ H bedeuten, R⁵ Cyclopropyl, 2-(C(=O)O-Ethyl)-cyclopropyl, Cyclohexyl, 2-Pyridinyl, C(=O)Phenyl, CO₂H oder CO₂Ethyl, R⁶ H, Br, CO₂Ethyl oder Methyl bedeutet, R⁷ H, NH₂, OH oder Methyl bedeutet und R⁹ H, Cl, OH oder CF₃ bedeutet oder R⁶ und R⁹ gemeinsam T bilden, wobei T γ'-N=C(CO₂H)-CH=N-ε' bedeutet.

5. Verbindungen der allgemeinen Struktur I nach einem der Ansprüche 3 und 4 oder ihre pharmazeutisch annehmbaren Salze, wobei die Verbindungen ausgewählt sind aus:
7-Brom-4-(4-methoxy-phenyl)-3,4-dihydro-2H-pyrimido[1,2-a]pyrimidin-2-carbonsäureethytester,
7-Brom-4-(3,4-dimethoxy-phenyl)-3,4-dihydro-2H-pyrimido[1,2-a]pyrimidin-2-carbonsäure,
7-Brom-4-(4-methoxy-phenyl)-3,4-dihydro-2H-pyrimido[1,2-a]pyrimidin-2-carbonsäure,
7-Brom-4-(4-hydroxy-phenyl)-3-methyl-3,4-dihydro-2H-pyrimido[1,2-a]pyrimidin-2-carbonsäureethylester,
2-Brom-6,6a,7,11b-tetrahydro-4,5,11c-triaza-benzo[c]fluoren-6-carbonsäureethylester,
7-Brom-4-(4-methoxy-phenyl)-3-methyl-3,4-dihydro-2H-pyrimido[12-a]pyrimidin-2-carbonsäure,
7-Brom-4-(3,4-dimethoxy-phenyl)-3,4-dihydro-2H-pyrimido[1,2-a]pyrimidin-2-carbonsäure,
7-Brom-4-(4-methoxy-phenyl)-3,4-dihydro-2H-pyrimido[1,2-a]pyrimidin-2-carbonsäure,
[7-Brom-4-(2-hydroxy-ethoxy)-3,4-dihydro-2H-pyrimido[1,2-a]pyrimidin-2-yl]-phenyl-methanon,
[7-Brom-4-(4-hydroxy-3-methoxy-phenyl)-3-methyl-3,4-dihydro-2Hpyrimido[1,2-a]pyrimidin-2-yl]-phenyl-methanon,
[7-Brom-4-(4-methoxy-phenyl)-3-methyl-3,4-dihydro-2H-pyrimido[1,2-a]pyrimidin-2-yl]-phenyl-methanon,
[7-Brom-4-(4-hydroxy-phenyl)-3-methyl-3,4-dihydro-2H-pyrimido[1,2-a]pyrimidin-2-yl]-phenyl-methanon,
[7-Brom-4-(3,4-dimethoxy-phenyl)-3,4-dihydro-2H-pyrimido[1,2-a]pyrimidin-2-yl]-phenyl-methanon,
[7-Brom-4-(4-methooy-phenyl)-3,4-dihydro-2H-pyrimido[1,2-a]pyrimidin-2-yl]-phenyl-methanon,
2-(7-Brom-2-cyclopropyl-3,4-dihydro-2H-pyrimido[1,2-a]pyrimidin-4-yloxy)-ethanol,
2-(7-Brom-2-cyclohexyl-3,4-dihydro-2H-pyrimido[1,2-a]pyrimidin-4-yloxy)-ethanol,
4-(7-Brom-2-cyclohexyl-3-methyl-3,4-dihydro-2H-pyrimido[1,2-a]pyrimidin-4-yl)-phenol,
7-Brom-4-naphthalin-2-yl-3,4-dihydro-2H-pyrimido[1,2-a]pyrimidin-2-carbonsäureethylester,
7-Brom-4-m-tolyl-3,4-dihydro-2H-pyrimido[1,2-a]pyrimidin-2-carbonsäureethylester,
2-[7-Brom-4-(2,4-dimethyl-phenyl)-3,4-dihydro-2H-pyrimido[1,2-a]pyrimidin-2-yl]-cyclopropancarbonsäureethylester,
2-[7-Brom-4-(4-methoxy-phenyl)-3,4-dihydro-2H-pyrimido[1,2-a]pyrimidin-2-yl]-cyclopropancarbonsäureethylester,
7-Brom-4-phenylsulfanyl-2-pyridin-2-yl-3,4-dihydro-2H-pyrimido[1,2-a]pyrimidin,
2-Chlor-6-phenylsulfanyl-8-pyridin-2-yl-7,8-dihydro-6H-pyrimido[1,2-a]pyrimidin-4-yl-amin,
6-Phenylsulfanyl-8-pyridin-2-yl-7,8-dihydro-6H-pyrimido[1,2-a]pyrimidin-2,4-diol,
6-Phenylsulfanyl-8-pyridin-2-yl-2-trifluormethyl-7,8-dihydro-6Hpyrimido[1,2-a]pyrimidin-3-carbonsäureethylester,
3-Methyl-5,8-methano-9-pyridin-2-yl-5,8,8a,9-tetrahydro-4bH-1,4a,10-triaza-phenanthren-2,4-diol,
5,8-Methano-9-pyridin-2-yl-5,8,8a,9-tetrahydro-4bH-1,4a,10-triazaphenanthren-2,4-diol,
12-Hydroxyl-1,4-methano-5-pyridin-2-yl-1,4a,5,12b-tetrahydro-4H-6,7,8,11,12a-pentaaza-benzo[a]anthracen-10-carbonsäure,
3-Bromo-9-pyridin-2-yl-7,8,8a,9-tetrahydro-4bH-1,4a,10-triazaphenanthren.

6. Verbindungen der allgemeinen Struktur I nach einem der Ansprüche 1 und 2 oder ihre pharmazeutisch annehmbaren Salze, wobei Y CR⁸ und Z N bedeuten, einer der Reste R¹ und R² OR¹⁰, SR¹⁰, C₁₋₆-Alkyl oder Aryl bedeutet, R³ und R⁴ H oder C₁₋₆-Alkyl bedeutet, oder einer der Reste R¹ und R² zusammen mit einem der Reste R³ und R⁴ W bildet, wobei W 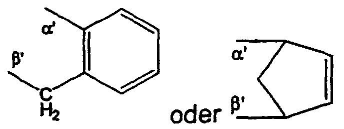 bedeutet, und die beiden anderen Reste von R¹, R², R³ und R⁴ H oder C₁₋₁₂-Alkyl bedeuten, R⁵ C₃₋₇-Cycloalkyl, Heteroaryl, C(=O)R¹¹, CO₂H oder CO₂R¹² bedeutet, R⁶ H, F, Cl, Br, CN, NO₂, C(=O)R¹⁴, C₁₋₆-Alkyl, CF₃ oder Aryl bedeutet, R⁷ H, F, Cl, Br, CN, NH₂, OH oder C₁₋₆-Alkyl bedeutet, R⁸ H, F, Cl, Br, CN, NO₂, O-(C₁₋₆-Alkyl)-Aryl, CO₂H, CONH₂ oder C₁₋₆-Alkyl bedeutet, R¹⁰ C₁₋₈-Alkyl oder Aryl bedeutet, R¹¹ Aryl bedeutet, R¹² C₁₋₆-Alkyl bedeutet und R¹⁴ OC₁₋₆-Alkyl bedeutet.

7. Verbindungen der allgemeinen Struktur I nach Anspruch 6 oder ihre pharmazeutisch annehmbaren Salze, wobei einer der Reste R¹ und R² O-(CH₂)₂-OH, S-Phenyl, Methyl, Phenyl, 2,4-Dimethyl-phenyl, 4-Hydroxyphenyl, 4-Methoxyphenyl, 4-Hydroxy-3-methoxy-phenyl oder 3,4-Dimethoxy-phenyl bedeutet, einer der Reste R³ und R⁴ H oder Methyl bedeutet, oder einer der Reste R¹ und R² zusammen mit einem der Reste R³ und R⁴ W bildet, wobei W 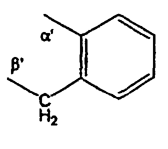 oder 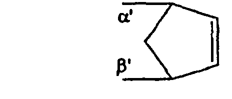 bedeutet, und die beiden anderen Reste von R¹, R², R³ und R⁴ H bedeuten, R⁵ Cyclopropyl, 2-(C(=O)O-Ethyl)-cyclopropyl, Cyclohexyl, 2-Pyridinyl, 5-Methyl-furan-2-yl, 5-Nitro-furan-2-yl, C(=O)Phenyl, CO₂H oder CO₂Ethyl, R⁶ H, CI, CN oder Phenyl bedeutet, R⁷ H, NH₂ oder CN bedeutet und R⁸ H, CI, CN, CO₂H oder CONH₂ bedeutet, sowie ihre pharmazeutisch annehmbaren Salze.

8. Verbindungen der allgemeinen Struktur I nach einem der Ansprüche 6 und 7 oder ihre pharmazeutisch annehmbaren Salze, wobei die Verbindungen ausgewählt sind aus:
4-(4-Methoxy-phenyl)-3-methyl-2-(5-nitro-furan-2-yl)-3,4-dihydro-2Hpyrazino[1,2-a]pyrimidin,
2-[4-(4-Methoxy-phenyl)-3-methyl-3,4-dihydro-2H-pyrazino[1,2-a]pyrimidin-2-yl]-cyclopropancarbonsäureethylester,
4-(4-Methoxy-phenyl)-3-methyl-2-(5-methyl-furan-2-yl)-3,4-dihydro-2H-pyrazino[1,2-a]pyrimidin,
4-(3,4-Dimethoxy-phenyl)-3,4-dihydro-2H-pyrazino[1,2-a]pyrimidin-2-carbonsäureethylester,
4-(4-Methoxy-phenyl)-3,4-dihydro-2H-pyrazino[1,2-a]pyrimidin-2-carbonsäure ethylester,
4-(3,4-Dimethoxy-phenyl)-3,4-dihydro-2H-pyrazino[1,2-a]pyrimidin-2-carbonsäure,
4-(4-Methoxy-phenyl)-3,4-dihydro-2H-pyrazino[1,2-a]pyrimidin-2-carbonsäure,
4-(4-Methoxy-phenyl)-2-(5-nitro-furan-2-yl)-3,4-dihydro-2Hpyrazino[1,2-a]pyrimidin,
2-[4-(2,4-Dimethyl-phenyl)-3,4-dihydro-2H-pyrazino[1,2-a]pyrimidin-2-yl]-cyclopropancarbonsäureethylester,
2-[4-(4-Methoxy-phenyl)-3,4-dihydro-2H-pyrazino[1,2-a]pyrimidin-2-yl]-cyclopropancarbonsäureethylester,
4-(4-Hydroxy-phenyl)-3-methyl-3,4-dihydro-2H-pyrazino[1,2-a]pyrimidin-2-carbonsäureethylester,
6,6a,7,11 b-Tetrahydro-3,5,11c-triaza-benzo[c]fluorene-6-carbonsäureethylester,
4-Phenylsulfanyl-3,4-dihydro-2H-pyrazino[1,2-a]pyrimidin-2-carbonsäureethylester,
4-Phenylsulfanyl-3,4-dihydro-2H-pyrazino[1,2-a]pyrimidin-2-carbonsäure,
4-(4-Methoxy-phenyl)-3-methyl-3,4-dihydro-2H-pyrazino[1,2-a]pyrimidin-2-carbonsäure,
4-(3,4-Dimethoxy-phenyl)-3,4-dihydro-2H-pyrazino[1,2-a]pyrimidin-2-carbonsäure,
4-(4-Methoxy-phenyl)-3,4-dihydro-2H-pyrazino[1,2-a]pyrimidin-2-carbonsäure,
[4-(2-Hydroxy-ethoxy)-3,4-dihydro-2H-pyrazino[1,2-a]pyrimidin-2-yl]-phenyl-methanon,
[4-(4-Hydroxy-3-methoxy-phenyl)-3-methyl-3,4-dihydro-2Hpyrazino[1,2-a]pyrimidin-2-yl]-phenyl-methanon,
[4-(4-Methoxy-phenyl)-3-methyl-3,4-dihydro-2H-pyrazino[1,2-a]pyrimidin-2-yl]-phenyl-methanon,
[4-(4-Hydroxy-phenyl)-3-methyl-3,4-dihydro-2H-pyrazino[1,2-a]pyrimidin-2-yl]-phenyl-methanon,
2-Cyclopropyl-4-(3,4-dimethoxy-phenyl)-3,4-dihydro-2H-pyrazino[1,2-a]pyrimidin,
2-Cyclopropyl-4-(4-methoxy-phenyl)-3,4-dihydro-2H-pyrazino[1,2-a]pyrimidin,
2-(2-Cyclohexyl-3,4-dihydro-2H-pyrazino[1,2-a]pyrimidin-4-yloxy)-ethanol,
2-Cyclohexyl-4-(4-methoxy-phenyl)-3,4-dihydro-2H-pyrazino[1,2-a]pyrimidin,
4-Methyl-2-(5-nitro-furan-2-yl)-4-phenyl-3,4-dihydro-2H-pyrazino[1,2-a]pyrimidin,
2-(4-Methyl-4-phenyl-3,4-dihydro-2H-pyrazino[1,2-a]pyrimidin-2-yl)-cyclopropancarbonsäureethylester,
4-Phenyl-2-pyridin-2-yl-3,4-dihydro-2H-pyrazino[1,2-a]pyrimidin,
4-Phenyl-2-pyridin-2-yl-3,4-dihydro-2H-pyrazino[1,2-a]pyrimidin-9-carbonsäure,
6-Amino-7-chlor-4-phenyl-2-pyridin-2-yl-3,4-dihydro-2H-pyrazino[1,2-a]pyrimidin-9-carbonsäureamid,
2-Phenylsulfanyl-3-pyridin-2-yl-2,3-dihydro-1H-pyrimido[1,2-a]chinolin-10-ol,
4-Phenylsulfanyl-2-pyridin-2-yl-3,4-dihydro-2H-pyrazino[1,2-a]pyrimidin-9-carbonsäure,
6-Amino-7-chlor-4-phenylsulfanyl-2-pyridin-2-yl-3,4-dihydro-2Hpyrazino[1,2-a]pyrimidin-9-carbonsäureamid,
7-Phenyl-4-phenylsulfanyl-2-pyridin-2-yl-3,4-dihydro-2H-pyrazino[1,2-a]pyrimidin-9-carbonitril,
5,8-Methano-9-pyridin-2-yl-5,8,8a,9-tetrahydro-4bH-2,4a,10-triazaphenanthren,
1-Chlor-5,8-methano-9-pyridin-2-yl-5,8,8a,9-tetrahydro-4bH-2,4a,10-triaza-phenanthren-3,4-dicarbonitril,
sowie ihre pharmazeutisch annehmbaren Salze.

9. Verfahren zur Herstellung von Verbindungen der allgemeinen Struktur I nach einem der Ansprüche 1 bis 8 und ihrer pharmazeutisch annehmbaren Salze, 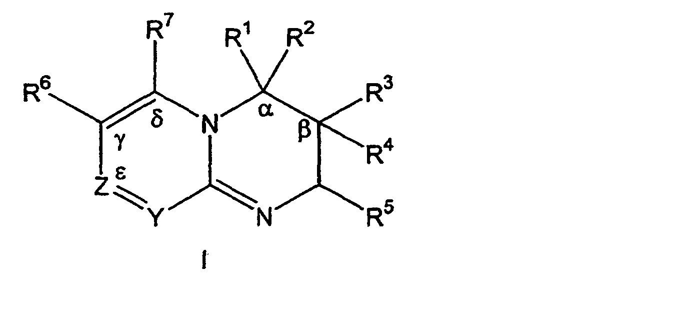 worin Y, Z und R¹ bis R⁷ wie in Anspruch 1 definiert sind,
**dadurch gekennzeichnet, daß**
ein Heteroarylamin der allgemeinen Struktur II 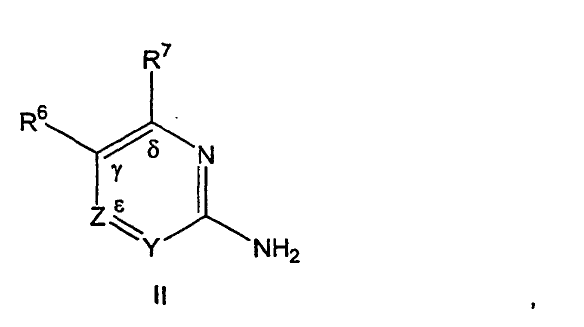 worin Y, Z, R⁶ und R⁷ wie oben definiert sind, mit der Maßgabe, daß, wenn R⁶ und R⁷ das wie oben definierte Q bilden, das mit γ' gekennzeichnete Ende von Q mit dem γ-Kohlenstoffatom des Heteroarylamins der allgemeinen Struktur II und das mit δ' gekennzeichnete Ende von Q mit dem δ-Kohlenstoffatom des Heteroarylamins der allgemeinen Struktur II verbunden ist, und daß, wenn R⁶ und R⁹ das wie oben definierte T bilden, das mit γ' gekennzeichnete Ende mit dem γ-Kohlenstoffatom des Heteroarylamins der allgemeinen Struktur II und das mit ε' gekennzeichnete Ende mit dem ε-Kohlenstoffatom des Heteroarylamins der allgemeinen Struktur II verbunden ist,
mit einem Aldehyd der allgemeinen Struktur III 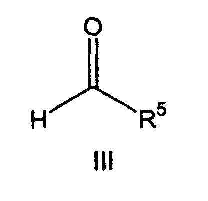 worin R⁵ wie oben definiert ist,
und einem Olefin der allgemeinen Struktur IV 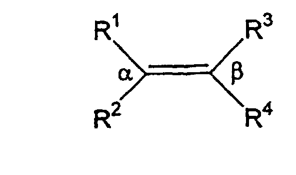 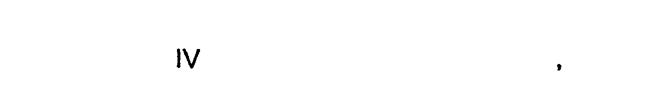 worin R¹, R², R³ und R⁴ wie oben definiert sind, mit der Maßgabe, daß wenn einer der Reste R¹ und R² zusammen mit einem der Reste R³ und R⁴ W bildet, das mit α' gekennzeichnete Ende von W mit dem α-Kohlenstoffatom des Olefins der allgemeinen Struktur IV und das mit β' gekennzeichnete Ende von W mit dem β-Kohtenstoffatom des Olefins der allgemeinen Struktur IV verbunden ist, in Gegenwart einer Säure umgesetzt wird.

10. Verfahren nach Anspruch 9, **dadurch gekennzeichnet, daß** die Umsetzung des Heteroarylamins der allgemeinen Struktur II mit dem Aldehyd der allgemeinen Struktur III und dem Olefin der allgemeinen Struktur IV in einem Eintopf-Verfahren durchgeführt wird.

11. Verfahren nach einem der Ansprüche 9 und 10, **dadurch gekennzeichnet, daß** die Säure Trifluoressigsäure ist.

12. Verfahren nach einem der Ansprüche 9 bis 11, **dadurch gekennzeichnet, daß** die Umsetzung in einem organischen Lösungsmittel bei einer Temperatur von 0 bis 100 °C und einer Reaktionszeit von 0,25 bis 12 h durchgeführt wird.

13. Verfahren nach einem der Ansprüche 9 bis 12, **dadurch gekennzeichnet, daß** die Reaktion bei einer Temperatur von 15 bis 40 °C durchgeführt wird.

14. Verfahren nach einem der Ansprüche 9 bis 13, **dadurch gekennzeichnet, daß** das organische Lösungsmittel Acetonitril ist.

15. Substanzbibliothek, enthaltend mindestens eine Verbindung der allgemeinen Struktur I nach einem der Ansprüche 1 bis 8.

16. Arzneimittel, enthaltend mindestens eine Verbindung der allgemeinen Struktur I oder eines ihrer pharmazeutisch annehmbaren Salze nach einem der Ansprüche 1 bis 8.

17. Verwendung einer Verbindung der allgemeinen Struktur I nach einem der Ansprüche 1 bis 8 oder eines ihrer pharmazeutisch annehmbaren Salze zur Herstellung eines Arzneimittels zur Behandlung von Schmerz.

18. Verwendung einer Verbindung der allgemeinen Struktur I nach einem der Ansprüche 1 bis 8 oder eines ihrer pharmazeutisch annehmbaren Salze zur Herstellung eines Arzneimittels zur Behandlung von Haminkontinenz, Juckreiz, Tinnitus aurium und/oder Diarrhoe.

19. Pharmazeutische Zusammensetzung, die mindestens eine Verbindung der allgemeinen Struktur I oder eines ihrer pharmazeutisch annehmbaren Salze nach einem der Ansprüche 1 bis 8 sowie mindestens einen pharmazeutischen Hilfsstoff enthält.

## Claims

1. Compound of general structure I 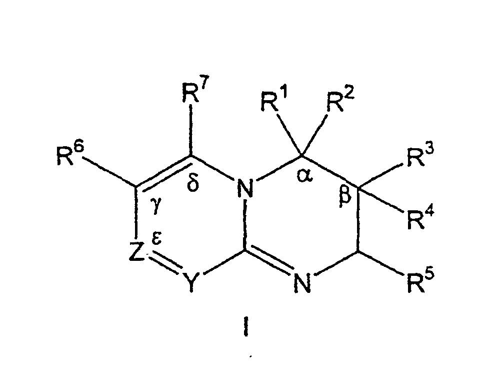 wherein
Y represents CR⁸ and
Z represents N or
Y represents N and
Z represents CR⁹,
R¹ and R² independently of one another represent H, OR¹⁰, SH, SR¹⁰, C₁₋₁₂ alkyl, wherein alkyl is straight-chained or branched and saturated or unsaturated and unsubstituted or singly or multiply substituted, C₃₋₈ cycloalkyl, wherein cycloalkyl is saturated or unsaturated and unsubstituted or singly or multiply substituted, heterocyclyl, wherein heterocyclyl is 3-, 4-, 5-, 6- or 7-membered and is saturated or unsaturated and unsubstituted or singly or multiply substituted, aryl, wherein aryl is unsubstituted or singly or multiply substituted, heteroaryl, wherein heteroaryl is unsubstituted or singly or multiply substituted, (C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted, or (C₁₋₆ alkyl)-heteroaryl, wherein C₁₋₆ alkyl is straight-chained or branched and saturated or unsaturated and unsubstituted or singly or multiply substituted and heteroaryl is unsubstituted or singly or multiply substituted, wherein one of R¹ and R² is H and the other radical from R¹ and R² is not H with the proviso that if one of R¹ and R² represents aryl the other radical from R¹ and R² represents H or C₁₋₁₂ alkyl,
R³ and R⁴ independently of one another represent H, C₁₋₁₂ alkyl, wherein C₁₋₁₂ alkyl alkyl is straight-chained or branched and saturated or unsaturated and unsubstituted or singly or multiply substituted, C₃₋₈ cycloalkyl, wherein cycloalkyl is saturated or unsaturated and unsubstituted or singly or multiply substituted, aryl, wherein aryl is unsubstituted or singly or multiply substituted, heteroaryl, wherein heteroaryl is unsubstituted or singly or multiply substituted, (C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted, or (C₁₋₆ alkyl)-heteroaryl, wherein C₁₋₆ alkyl is straight-chained or branched and saturated or unsaturated and unsubstituted or singly or multiply substituted and heteroaryl is unsubstituted or singly or multiply substituted, wherein at least one of R³ and R⁴ is H, or one of the radicals R¹ or R² together with one of the radicals R³ or R⁴ forms W, wherein W represents α'-(CH₂)ₙ-β' where n = 3, 4, 5, 6, 7, 8, 9 or 10, α'-CH=CH-CH₂-β', α'-CH=CH-CH₂-CH₂-β', α'-CH₂-CH=CH-CH₂-β', α'-CH₂-CH₂-CH=CH-CH₂-CH₂-β', α'-O-(CH₂)ₙ-β' where n = 2, 3, 4, 5 or 6, 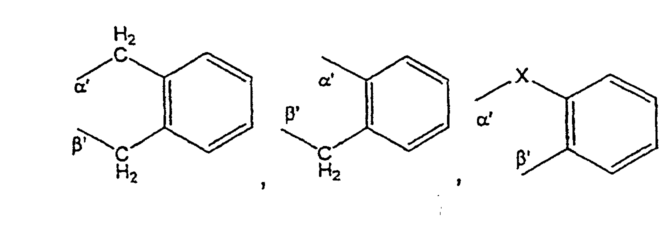 where X = CH₂, O or S, 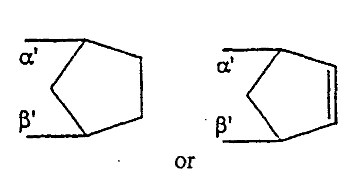 and the end of W denoted by α' is joined to the α-carbon atom of the compound of general formula I and the end of W denoted by β' is joined to the β-carbon atom of the compound of general structure I, the other radical from R¹ and R² is H or C₁₋₁₂ alkyl, wherein alkyl is straight-chained or branched and saturated or unsaturated and unsubstituted or singly or multiply substituted, and the other radical from R³ and R⁴ is H or C₁₋₁₂ alkyl, wherein alkyl is straight-chained or branched and saturated or unsaturated and unsubstituted or singly or multiply substituted, .
R⁵ represents C₁₋₁₂ alkyl, wherein C₁₋₁₂ alkyl is straight-chained or branched and saturated or unsaturated and unsubstituted or singly or multiply substituted, C₃₋₈ cycloalkyl, wherein cycloalkyl is saturated or unsaturated and unsubstituted or singly or multiply substituted, heterocyclyl, wherein heterocyclyl is 3-, 4-, 5-, 6- or 7-membered and is saturated or unsaturated and unsubstituted or singly or multiply substituted, aryl, wherein aryl is unsubstituted or singly or multiply substituted, heteroaryl, wherein heteroaryl is unsubstituted or singly or multiply substituted, (C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted, or (C₁₋₆ alkyl)-heteroaryl, wherein C₁₋₆ alkyl is straight-chained or branched and saturated or unsaturated and unsubstituted or singly or multiply substituted and heteroaryl is unsubstituted or singly or multiply substituted, C(=O)R¹¹, CO₂H or CO₂R¹²,
R⁶, R⁷, R⁸ and R⁹ independently of one another represent H, F, Cl, Br, I, CN, NH₂, NH(C₁₋₆ alkyl), N(C₁₋₆ alkyl)₂, NH((C₁₋₆ alkyl)-aryl), N((C₁₋₆ alkyl)-aryl)₂, NH-aryl, N(aryl)₂, NHR¹³, NO₂, OH, SH, O-C₁₋₈ alkyl or S(O)ₚ-C₁₋₈ alkyl, wherein alkyl is straight-chained or branched and saturated or unsaturated and unsubstituted or singly or multiply substituted and p is 0, 1 or 2, O-aryl or S(O)_{q} aryl, wherein aryl is unsubstituted or singly or multiply substituted and q is 0, 1 or 2, O-(C₁₋₆ alkyl)-aryl or S(O)ᵣ-(C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted and r is 0, 1 or 2, CO₂H, C(=O)R¹⁴, C₁₋₁₂ alkyl, wherein alkyl is straight-chained or branched and saturated or unsaturated and unsubstituted or singly or multiply substituted, CF₃, C₃₋₈ cycloalkyl, wherein cycloalkyl is saturated or unsaturated and unsubstituted or singly or multiply substituted, heterocyclyl, wherein heterocyclyl is 3-, 4-, 5-, 6- or 7-membered and is saturated or unsaturated and unsubstituted or singly or multiply substituted, aryl, wherein aryl is unsubstituted or singly or multiply substituted, or heteroaryl, wherein heteroaryl is unsubstituted or singly or multiply substituted or R⁶ and R⁷ together form Q, wherein Q represents γ'-CR¹⁵=CR¹⁶-CR¹⁷=CR¹⁸-δ', the end of Q denoted by γ' is joined to the γ-carbon atom of the compound of general structure I and the end of Q denoted by δ' is joined to the δ-carbon atom of the compound of general structure I, and Y and Z are as defined above, or R⁶ and R⁹ together form T, wherein T represents γ'-CR¹⁹=CR²⁰-CR²¹=CR²²-ε' or γ'-N=CR²⁰-CR²¹=N-ε', the end of T denoted by γ' is joined to the γ-carbon atom of the compound of general structure I and the end of T denoted by ε' is joined to the ε-carbon atom of the compound of general structure I and R⁷ and R⁸ are as defined above,
R¹⁰ represents C₁₋₈ alkyl, wherein alkyl is straight-chained or branched and saturated or unsaturated and unsubstituted or singly or multiply substituted, C₃₋₈ cycloalkyl, wherein cycloalkyl is saturated or unsaturated and unsubstituted or singly or multiply substituted, heterocyclyl, wherein heterocyclyl is 3-, 4-, 5-, 6- or 7-membered and is saturated or unsaturated and unsubstituted or singly or multiply substituted, aryl, wherein aryl is unsubstituted or singly or multiply substituted, heteroaryl, wherein heteroaryl is unsubstituted or singly or multiply substituted, (C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted, or (C₁₋₆ alkyl)-heteroaryl, wherein C₁₋₆ alkyl is straight-chained or branched and saturated or unsaturated and unsubstituted or singly or multiply substituted and heteroaryl is unsubstituted or singly or multiply substituted,
R¹¹ represents NH₂, NH(C₁₋₆ alkyl), N(C₁₋₆ alkyl)₂, NH((C₁₋₆ alkyl)-aryl), N((C₁₋₆ alkyl)-aryl)₂, NH-aryl, N(aryl)₂, C₁₋₈ alkyl, wherein alkyl is straight-chained or branched and saturated or unsaturated and unsubstituted or singly or multiply substituted, C₃₋₈ cycloalkyl, wherein cycloalkyl is saturated or unsaturated and unsubstituted or singly or multiply substituted, aryl, wherein aryl is unsubstituted or singly or multiply substituted, heteroaryl, wherein heteroaryl is unsubstituted or singly or multiply substituted, (C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted, or (C₁₋₆ alkyl)-heteroaryl, wherein C₁₋₆ alkyl is straight-chained or branched and saturated or unsaturated and unsubstituted or singly or multiply substituted and heteroaryl is unsubstituted or singly or multiply substituted,
R¹² represents C₁₋₈ alkyl, wherein alkyl is straight-chained or branched and saturated or unsaturated and unsubstituted or singly or multiply substituted, C₃₋₈ cycloalkyl, wherein cycloalkyl is saturated or unsaturated and unsubstituted or singly or multiply substituted, (C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted,
R¹³ represents C(=O)CH₃, C(=O) phenyl or C(=O)O-t.-butyl(t-BOC),
R¹⁴ represents H, NH₂, NH(C₁₋₆ alkyl), N(C₁₋₆ alkyl)₂, NH((C₁₋₆ alkyl)-aryl), N((C₁₋₆ alkyl)-aryl)₂, NH-aryl, N(aryl)₂, C₁₋₈ alkyl, wherein alkyl is straight-chained or branched and saturated or unsaturated and unsubstituted or singly or multiply substituted, C₃₋₈ cycloalkyl, wherein cycloalkyl is saturated or unsaturated and unsubstituted or singly or multiply substituted, aryl, wherein aryl is unsubstituted or singly or multiply substituted, (C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted, OC₁₋₈ alkyl, wherein alkyl is straight-chained or branched and saturated or unsaturated and unsubstituted or singly or multiply substituted, OC₃₋₈ cycloalkyl wherein cycloalkyl is saturated or unsaturated and unsubstituted or singly or multiply substituted, O aryl, wherein aryl is unsubstituted or singly or multiply substituted, or O-(C₁₋₆ alkyl)-aryl, wherein C₁₋₆ alkyl is straight-chained or branched and saturated or unsaturated and unsubstituted or singly or multiply substituted and aryl is unsubstituted or singly or multiply substituted,
R¹⁵, R¹⁶, R¹⁷ and R¹⁸ independently of one another represent H, F, Cl, Br, I, OH, CN, C₁₋₈ alkyl, wherein alkyl is straight-chained or branched and saturated or unsaturated and unsubstituted or singly or multiply substituted, CO₂H, and
R¹⁹, R²⁰, R²¹ and R²² independently of one another represent H, F, Cl, Br, I, CN, OH, C₁₋₈ alkyl, wherein alkyl is straight-chained or branched and saturated or unsaturated and unsubstituted or singly or multiply substituted, or CO₂H,
and the pharmaceutically acceptable salts thereof.

2. Compounds of general structure I according to claim 1 in the form of their racemates, in the form of their pure enantiomers or in the form of blends of the enantiomers or diastereomers in any mixing ratio, and their pharmaceutically acceptable salts.

3. Compounds of general structure I according to either of claims 1 or 2 or their pharmaceutically acceptable salts, wherein Y represents N and Z represents CR⁹, one of the radicals R¹ and R² represents OR¹⁰, SR¹⁰, C₁₋₆ alkyl or aryl, one of the radicals R³ and R⁴ represents H or C₁₋₆ alkyl, or one of the radicals R¹ and R² together with one of the radicals R³ and R⁴ forms W, wherein W represents α'-CH=CH-CH₂-CH₂-β', 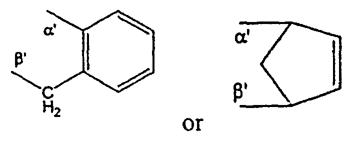 and the two other radicals from R¹, R², R³ and R⁴ represent H or C₁₋₁₂ alkyl, R⁵ represents C₃₋₇ cycloalkyl, heteroaryl, C(=O)R¹¹, CO₂H or CO₂R¹², R⁶ represents H, F, Cl, Br, CN, NO₂, C(=O)R¹⁴, C₁₋₆ alkyl, CF₃ or aryl, R⁷ represents H, F, Cl, Br, CN, NH₂, OH or C₁₋₆ alkyl, R⁹ represents H, OH, CF₃ or C₁₋₆ alkyl or R⁶ and R⁹ together form T, wherein T represents γ'-N=CR²⁰-CR²¹=N-ε', R¹⁰ represents C₁₋₈ alkyl or aryl, R¹¹ represents aryl, R¹² represents C₁₋₆ alkyl, R¹⁴ represents OC₁₋₆ alkyl, R²⁰ represents H or CH₂H and R²¹ represents H.

4. Compounds of general structure I according to claim 3 or their pharmaceutically acceptable salts, wherein one of the radicals R¹ and R² represents O-(CH₂)₂-OH, S-phenyl, phenyl, 3-methylphenyl, 2,4-dimethylphenyl, 4-hydroxyphenyl, 4-methoxyphenyl, 4-hydroxy-3-methoxyphenyl, 3,4-dimethoxyphenyl or 2-naphthyl, one of the radicals R³ and R⁴ represents H or methyl, or one of the radicals R¹ and R² together with one of the radicals R³ and R⁴ forms W, wherein W represents α'-CH=CH-CH₂-CH₂-β', 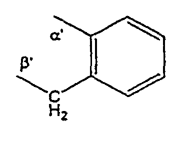 or 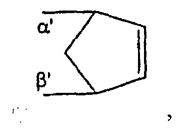 and the two other radicals from R¹, R², R³ and R⁴ represent H, R⁵ represents cyclopropyl, 2-(C(=O)O-ethyl)-cyclopropyl, cyclohexyl, 2-pyridinyl, C(=O) phenyl, CO₂H or CO₂ ethyl, R⁶ represents H, Br, CO₂ ethyl or methyl, R⁷ represents H, NH₂, OH or methyl and R⁹ represents H, Cl, OH or CF₃ or R⁶ and R⁹ together form T, wherein T represents γ'-N=C(CO₂H)-CH=N-ε'.

5. Compounds of general structure I according to either of claims 3 or 4 or their pharmaceutically acceptable salts, wherein the compounds are selected from:
7-bromo-4-(4-methoxy-phenyl)-3,4-dihydro-2H-pyrimido[1,2a]pyrimidine-2-carboxylic acid ethyl ester,
7-bromo-4-(3,4-dimethoxy-phenyl)-3,4-dihydro-2H-pyrimido[1,2a]pyrimidine-2-carboxylic acid,
7-bromo-4-(4-methoxy-phenyl)-3,4-dihydro-2H-pyrimido[1,2a]pyrimidine-2-carboxylic acid,
7-bromo-4-(4-hydroxy-phenyl)-3-methyl-3,4-dihydro-2H-pyrimido[1,2a]pyrimidine-2-carboxylic acid ethyl ester,
2-bromo-6,6a,7,11b-tetrahydro-4,5,11c-triaza-benzo[c]fluorene-6-carboxylic acid ethyl ester,
7-bromo-4-(4-methoxy-phenyl)-3-methyl-3,4-dihydro-2H-pyrimido[1,2a]pyrimidine-2-carboxylic acid,
7-bromo-4-(3,4-dimethoxy-phenyl)-3,4-dihydro-2H-pyrimido[1,2a]pyrimidine-2-carboxylic acid,
7-bromo-4-(4-methoxy-phenyl)-3,4-dihydro-2H-pyrimido[1,2a]pyrimidine-2-carboxylic acid,
[7-bromo-4-(2-hydroxy-ethoxy)-3,4-dihydro-2H-pyrimido[1,2a]pyrimidin-2-yl]-phenyl-methanone,
[7-bromo-4-(4-hydroxy-3-methoxy-phenyl)-3-methyl-3,4-dihydro-2H pyrimido[1,2a]pyrimidin-2-yl]-phenyl-methanone,
[7-bromo-4-(4-methoxy-phenyl)-3-methyl-3,4-dihydro-2H-pyrimido[1,2a]pyrimidin-2-yl]-phenyl-methanone,
[7-bromo-4-(4-hydroxy-phenyl)-3-methyl-3,4-dihydro-2H-pyrimido[1,2a]pyrimidin-2-yl]-phenyl-methanone,
[7-bromo-4-(3,4-dimethoxy-phenyl)-3,4-dihydro-2H-pyrimido[1,2a]pyrimidin-2-yl]-phenyl-methanone,
[7-bromo-4-(4-methoxy-phenyl)-3,4-dihydro-2H-pyrimido[1,2a]pyrimidin-2-yl]-phenyl-methanone,
2-(7-bromo-2-cyclopropyl-3,4-dihydro-2H-pyrimido[1,2a]pyrimidin-4yloxy)-ethanol,
2-(7-bromo-2-cyclohexyl-3,4-dihydro-2H-pyrimido[1,2a]pyrimidin-4yloxy)-ethanol,
4-(7-bromo-2-cyclohexyl-3-methyl-3,4-dihydro-2H-pyrimido[1,2a]pyrimidin-4-yl)-phenol,
7-bromo-4-naphthalen-2-yl-3,4-dihydro-2H-pyrimido[1,2a]pyrimidine-2-carboxylic acid ethyl ester,
7-bromo-4-m-tolyl-3,4-dihydro-2H-pyrimido[1,2a]pyrimidine-2 carboxylic acid ethyl ester,
2-[7-bromo-4-(2,4-dimethyl-phenyl)-3,4-dihydro-2H-pyrimido[1,2a]pyrimidin-2-yl]-cyclopropane carboxylic acid ethyl ester,
2-[7-bromo-4-(4-methoxy-phenyl)-3,4-dihydro-2H-pyrimido[1,2a]pyrimidin-2-yl]-cyclopropane carboxylic acid ethyl ester,
7-bromo-4-phenylsulphanyl-2-pyridin-2-yl-3,4-dihydro-2H-pyrimido[1,2-a]pyrimidine,
2-chloro-6-phenylsulphanyl-8-pyridin-2-yl-7,8-dihydro-6H-pyrimido[1,2a]pyrimidin-4-yl-amine,
6-phenylsulphanyl-8-pyridin-2-yl-7,8-dihydro-6H-pyrimido[1,220 a]pyrimidine-2,4-diol,
6-phenylsulphanyl-8-pyridin-2-yl-2-trifluoromethyl-7,8-dihydro-6H-pyrimido[1,2a]pyrimidine-3-carboxylic acid ethyl ester,
3-methyl-5,8-methano-9-pyridin-2-yl-5,8,8a,9-tetrahydro-4bH-1,4a,10-triazaphenanthrene-2,4-diol,
5,8-methano-9-pyridin-2-yl-5,8,8a,9-tetrahydro-4bH-1,4a,10-triazaphenanthrene-2,4-diol,
12-hydroxyl-1,4-methano-5-pyridin-2-yl-1,4a,5,12b-tetrahydro-4H-6,7,8,11,12a-pentaaza-benzo[a]anthracene-10-carboxylic acid,
3-bromo-9-pyridin-2-yl-7,8,8a,9-tetrahydro-4bH-1,4a,10-triaza-phenanthrene.

6. Compounds of general structure I according to either of claims 1 or 2 or their pharmaceutically acceptable salts, wherein Y represents CR⁸ and Z represents N, one of the radicals R¹ and R² represents OR¹⁰, SR¹⁰, C₁₋₆ alkyl or aryl, R³ and R⁴ represents H or C₁₋₆ alkyl, or one of the radicals R¹ and R² together with one of the radicals R³ and R⁴ forms W, wherein W represents 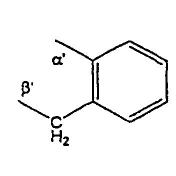 or 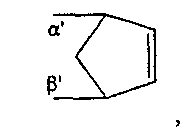 and the two other radicals from R¹, R², R³ and R⁴ represent H or C₁₋₁₂ alkyl, R⁵ represents C₃₋₇ cycloalkyl, heteroaryl, C(=O)R¹¹, CO₂H or CO₂R¹², R⁶ represents H, F, Cl, Br, CN, NO₂, C(=O)R¹⁴, C₁₋₆ alkyl, CF₃ or aryl, R⁷ represents H, F, Cl, Br, CN, NH₂, OH or C₁₋₆ alkyl, R⁸ represents H, F, Cl, Br, CN, NO₂, O-(C₁₋₆ alkyl)-aryl, CO₂H, CONH₂ or C₁₋₆ alkyl, R¹⁰ represents C₁₋₈ alkyl or aryl, R¹¹ represents aryl, R¹² represents C₁₋₆ alkyl and R¹⁴ represents OC₁₋₆ alkyl.

7. Compounds of general structure I according to claim 6 or their pharmaceutically acceptable salts, wherein one of the radicals R¹ and R² represents O-(CH₂)₂-OH, S-phenyl, phenyl, 3-methylphenyl, 2,4-dimethylphenyl, 4-hydroxyphenyl, 4-methoxyphenyl, 4-hydroxy-3-methoxyphenyl or 3,4-dimethoxyphenyl, one of the radicals R³ and R⁴ represents H or methyl, or one of the radicals R¹ and R² together with one of the radicals R³ and R⁴ forms W, wherein W represents 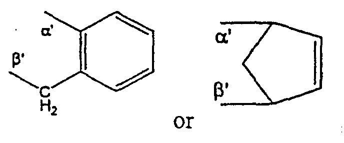 and the two other radicals from R¹, R², R³ and R⁴ represent H, R⁵ represents cyclopropyl, 2-(C(=O)O-ethyl)-cyclopropyl, cyclohexyl, 2-pyridinyl, 5-methyl-furan-2-yl, 5-nitro-furan-2-yl, C(=O) phenyl, CO₂H or CO₂ ethyl, R⁶ represents H, Cl, CN or phenyl, R⁷ represents H, NH₂ or CN and R⁸ represents H, Cl, CN, CO₂H or CONH₂, and their pharmaceutically acceptable salts.

8. Compounds of general structure I according to either of claims 6 or 7 or their pharmaceutically acceptable salts, wherein the compounds are selected from:
4-(4-methoxy-phenyl)-3-methyl-2-(5-nitro-furan-2-yl)-3,4-dihydro-2H-pyrazino[1,2a]pyrimidine,
2-[4-(4-methoxy-phenyl)-3-methyl-3,4-dihydro-2H-pyrazino[1,2a]pyrimidin-2-yl]-cyclopropane carboxylic acid ethyl ester,
4-(4-methoxy-phenyl)-3-methyl-2-(5-methyl-furan-2-yl)-3,4-dihydro-2H-pyrazino[1,2a]pyrimidine,
4-(3,4-dimethoxy-phenyl)-3,4-dihydro-2H-pyrazino[1,2a]pyrimidine-2-carboxylic acid ethyl ester,
4-(4-methoxy-phenyl)-3,4-dihydro-2H-pyrazino[1,2a]pyrimidine-2-carboxylic acid ethyl ester,
4-(3,4-dimethoxy-phenyl)-3,4-dihydro-2H-pyrazino[1,2a]pyrimidine-2-carboxylic acid,
4-(4-methoxy-phenyl)-3,4-dihydro-2H-pyrazino[1,2a]pyrimidine-2-carboxylic acid,
4-(4-methoxy-phenyl)-2-(5-nitro-furan-2-yl)-3,4-dihydro-2H-pyrazino[1,2a]pyrimidine,
2-[4-(2,4-dimethyl-phenyl)-3,4-dihydro-2H-pyrazino[1,2a]pyrimidin-2-yl]-cyclopropane carboxylic acid ethyl ester,
2-[4-(4-methoxy-phenyl)-3,4-dihydro-2H-pyrazino[1,2a]pyrimidin-2-yl]cyclopropane carboxylic acid ethyl ester,
4-(4-hydroxy-phenyl)-3-methyl-3,4-dihydro-2H-pyrazino[1,2a]pyrimidine-2-carboxylic acid ethyl ester,
6,6a,7,11 b-tetrahydro-3,5,11 c-triaza-benzo[c]fluorene-6-carboxylic acid ethyl ester,
4-phenylsulphanyl-3,4-dihydro-2H-pyrazino[1,2a]pyrimidine-2-carboxylic acid ethyl ester,
4-phenylsulphanyl-3,4-dihydro-2H-pyrazino[1,2a]pyrimidine-2-carboxylic acid,
4-(4-methoxy-phenyl)-3-methyl-3,4-dihydro-2H-pyrazino[1,2a]pyrimidine-2-carboxylic acid,
4-(3,4-dimethoxy-phenyl)-3,4-dihydro-2H-pyrazino[1,2a]pyrimidine-2-carboxylic acid,
4-(4-methoxy-phenyl)-3,4-dihydro-2H-pyrazino[1,2a]pyrimidine-2-carboxylic acid,
[4-(2-hydroxy-ethoxy)-3,4-dihydro-2H-pyrazino[1,2a]pyrimidin-2-yl]-phenyl-methanone,
[4-(4-hydroxy-3-methoxy-phenyl)-3-methyl-3,4-dihydro-2H-pyrazino[1,2a]pyrimidin-2-yl]-phenyl-methanone,
[4-(4-methoxy-phenyl)-3-methyl-3,4-dihydro-2H-pyrazino[1,2a]pyrimidin-2-yl]-phenyl-methanone,
[4-(4-hydroxy-phenyl)-3-methyl-3,4-dihydro-2H-pyrazino[1,2a]pyrimidin-2-yl]-phenyl-methanone, 2-cyclopropyl-4-(3,4-dimethoxy-phenyl)-3,4-dihydro-2H-pyrazino[1,2a]pyrimidine,
2-cyclopropyl-4-(4-methoxy-phenyl)-3,4-dihydro-2H-pyrazino[1,2a]pyrimidine,
2-(2-cyclohexyl-3,4-dihydro-2H-pyrazino[1,2a]pyrimidin-4-yloxy)ethanol,
2-cyclohexyl-4-(4-methoxy-phenyl)-3,4-dihydro-2H-pyrazino[1,2a]pyrimidine,
4-methyl-2-(5-nitro-furan-2-yl)-4-phenyl-3,4-dihydro-2H-pyrazino[1,2a]pyrimidine,
2-(4-methyl-4-phenyl-3,4-dihydro-2H-pyrazino[1,2a]pyrimidin-2-yl)cyclopropane carboxylic acid ethyl ester,
4-phenyl-2-pyridin-2-yi-3,4-dihydro-2H-pyrazino[1,2a]pyrimidine,
4-phenyl-2-pyridin-2-yl-3,4-dihydro-2H-pyrazino[1,2a]pyrimidine-9-carboxylic acid,
6-amino-7-chloro-4-phenyl-2-pyridin-2-yl-3,4-dihydro-2H-pyrazino[1,2a]pyrimidine-9-carboxylic acid amide,
2-phenylsulphanyl-3-pyridin-2-yl-2,3-dihydro-1H-pyrimido[1,2a]quinolin-10-ol,
4-phenylsulphanyl-2-pyridin-2-yl-3,4-dihydro-2H-pyrazino[1,2a]pyrimidine-9-carboxylic acid,
6-amino-7-chloro-4-phenylsulphanyl-2-pyridin-2-yl-3,4-dihydro-2H-pyrazino[1,2a]pyrimidine-9-carboxylic acid amide,
7-phenyl-4-phenylsulphanyl-2-pyridin-2-yl-3,4-.dihydro-2H-pyrazino[1,2a]pyrimidine-9-carbonitrile,
5,8-methano-9-pyridin-2-yl-5,8,8a,9-tetrahydro-4bH-2,4a,10-triazaphenanthrene, 1-chloro-5,8-methano-9-pyridin-2-yl-5,8,8a,9-tetrahydro-4bH-2,4a,10-triazaphenanthrene-3,4-dicarbonitrile,
and their pharmaceutically acceptable salts.

9. Method for producing compounds of general structure I according to any of claims 1 to 8 and their pharmaceutically acceptable salts, 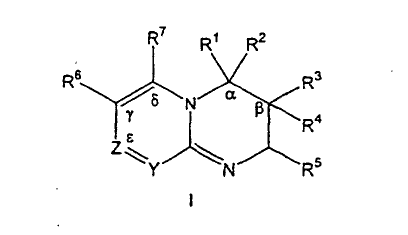 wherein Y, Z and R¹ to R⁷ are as defined in claim 1,
**characterised in that**
a heteroarylamine of general structure II 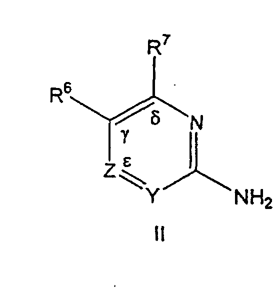 wherein Y, Z, R⁶ and R⁷ are as defined above, with the proviso that if R⁶ and R⁷ form Q as defined above, the end of Q denoted by γ' is joined to the γ-carbon atom of the heteroarylamine of general structure II and the end of Q denoted by δ' is joined to the δ-carbon atom of the heteroarylamine of general structure II, and that if R⁶ and R⁹ form T as defined above, the end denoted by γ' is joined to the γ-carbon atom of the heteroarylamine of general structure II and the end denoted by ε' is joined to the ε-carbon atom of the heteroarylamine of general structure II,
is reacted with an aldehyde of general structure III 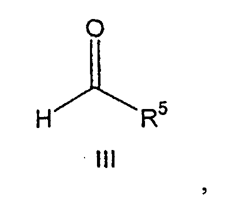 wherein R⁵ is as defined above,
and an olefin of general structure IV 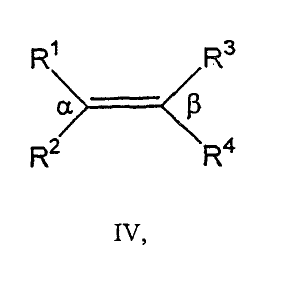 wherein R¹, R², R³ and R⁴ are as defined above, with the proviso that if one of the radicals R¹ and R² together with one of the radicals R³ and R⁴ forms W, the end of W denoted by α' is joined to the α-carbon atom of the olefin of general structure IV and the end of W denoted by β' is joined to the β-carbon atom of the olefin of general structure IV, in the presence of an acid.

10. Method according to claim 9, **characterised in that** the heteroarylamine of general structure II is reacted with the aldehyde of general structure III and the olefin of general structure IV by a one pot method.

11. Method according to either of claims 9 or 10, **characterised in that** the acid is trifluoroacetic acid.

12. Method according to any of claims 9 to 11, **characterised in that** the reaction is carried out in an organic solvent at a temperature of 0 to 100°C and a reaction time of 0.25 to 12 h.

13. Method according to any of claims 9 to 12, **characterised in that** the reaction is carried out at a temperature of 15 to 40°C.

14. Method according to any of claims 9 to 13, **characterised in that** the organic solvent is acetonitrile.

15. Substance library containing at least one compound of general structure I according to any of claims 1 to 8.

16. Pharmaceutical preparation containing at least one compound of general structure I or one of its pharmaceutically acceptable salts according to any of claims 1 to 8.

17. Use of a compound of general structure I according to any of claims 1 to 8 or one of its pharmaceutically acceptable salts for producing a pharmaceutical composition to treat pain.

18. Use of a compound of general structure I according to any of claims 1 to 8 or one of its pharmaceutically acceptable salts for producing a pharmaceutical preparation to treat urinary incontinence, itching, tinnitus aurium and/or diarrhoea.

19. Pharmaceutical composition containing at least one compound of general structure I or one of its pharmaceutically acceptable salts according to any of claims 1 to 8 and at least one pharmaceutical auxiliary.

## Revendications

1. Composé de formule générale I 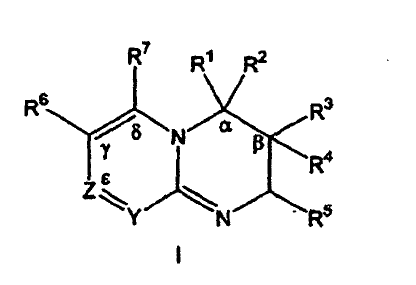 dans laquelle
Y représente CR⁸ et
Z représente N, ou bien
Y représente N et
Z représente CR⁹,
R¹ et R² représentent indépendamment l'un de l'autre H, un groupe OR¹⁰, SH, SR¹⁰, un reste alkyle en C₁ à C₁₂, ce reste alkyle étant linéaire ou ramifié et saturé ou non saturé et non substitué ou substitué une ou plusieurs fois, un reste cycloalkyle en C₃ à C₈, ce reste cycloalkyle étant saturé ou non saturé et non substitué ou substitué une ou plusieurs fois, un reste hétérocyclyle, ce reste hétérocyclyle ayant 3, 4, 5, 6 ou 7 chaînons et étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₆)-aryle, la partie alkyle en C₁ à C₆ étant linéaire ou ramifiée et saturée ou non saturée et non substituée ou substituée une ou plusieurs fois et la partie aryle étant non substituée ou substituée une ou plusieurs fois, ou bien un reste (alkyle en C₁ à C₆)-hétéroaryle, la partie alkyle en C₁ à C₆ étant linéaire ou ramifiée et saturée ou non saturée et non substituée ou substituée une ou plusieurs fois et la partie hétéroaryle étant non substituée ou substituée une ou plusieurs fois, l'un des restes R¹ et R² représentant H et l'autre ne représentant pas H, sous réserve que lorsque l'un de R¹ et R² désigne un reste aryle, l'autre de ces restes représente H ou un reste alkyle en C₁ à C₁₂,
R³ et R⁴ représentent, indépendamment l'un de l'autre H, un reste alkyle en C₁ à C₁₂, ce reste alkyle en C₁ à C₁₂ étant linéaire ou ramifié et saturé ou non saturé et étant non substitué ou substitué une ou plusieurs fois, un reste cycloalkyle en C₃ à C₈, ce reste cycloalkyle étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois, ou un reste (alkyle en C₁ à C₆)-hétéroaryle, la partie alkyle en C₁ à C₆ étant linéaire ou ramifiée et saturée ou non saturée et étant non substituée ou substituée une ou plusieurs fois et la partie hétéroaryle étant non substituée ou substituée une ou plusieurs fois, l'un au moins de R³ et R⁴ représentant H, ou bien
l'un des restes R¹ ou R² forme un groupe W conjointement avec les restes R³ ou R⁴, W étant un groupe α'-(CH₂)ₙ-β' dans lequel n a la valeur 3, 4, 5, 6, 7, 8, 9 ou 10, α'-CH=CH-CH₂-β', α'-CH=CH-CH₂-CH₂-β', α-CH₂-CH=CH-CH₂-β', α'-CH₂-CH₂-CH=CH-CH₂-CH₂-β', α-O-(CH₂)ₙ-β' où n a la valeur 2, 3, 4, 5 ou 6, 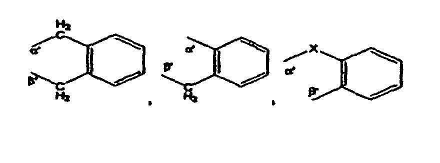 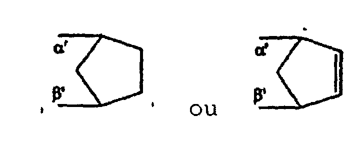 X représente CH₂, O ou S, et l'extrémité de W désignée par α' étant liée à l'atome α de carbone du composé de formule générale I et l'extrémité de W désignée par β' étant liée à l'atome β de carbone du composé de formule générale I, l'autre des restes R¹ et R² représente H ou un reste alkyle en C₁ à C₁₂, ce reste alkyle étant linéaire ou ramifié et saturé ou non saturé et étant non substitué ou substitué une ou plusieurs fois, et l'autre des restes R³ et R⁴ représente H ou un reste alkyle en C₁ à C₁₂, ce reste alkyle étant linéaire ou ramifié et saturé ou non saturé et étant non substitué ou substitué une ou plusieurs fois,
R⁵ est un reste alkyle en C₁ à C₁₂, ce reste alkyle en C₁ à C₁₂ étant linéaire ou ramifié et saturé ou non saturé et étant non substitué ou substitué une ou plusieurs fois, un reste cycloalkyle en C₃ à C₈, ce reste cycloalkyle étant saturé ou non saturé et non substitué ou substitué une ou plusieurs fois, un reste hétérocyclyle, ce reste hétérocyclyle ayant 3, 4, 5, 6 ou 7 chaînons et étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois, un reste (alkyle en C₁ à C₆)-hétéroaryle, la partie alkyle en C₁ à C₆ étant linéaire ou ramifiée et saturée ou non saturée et étant non substituée ou substituée une ou plusieurs fois et la partie hétéroaryle étant non substituée ou substituée une ou plusieurs fois, un groupe C(=O)R¹¹, CO₂H ou CO₂R¹²,
R⁶, R⁷, R⁸ et R⁹ représentent, indépendamment les uns des autres, H, F, Cl, Br, I, CN, un groupe NH₂, NH-(alkyle en C₁ à C₆), N- (alkyle en C₁ à C₆)₂, NH- ( (alkyle en C₁ à C₆)-aryle), N-((alkyle en C₁ à C₆)-aryle)₂, NH-aryle, N-(aryle)₂, NHR¹³, NO₂, OH, SH, O-(alkyle en C₁ à C₈) ou S(O)ₚ-(alkyle en C₁ à C₈), la partie alkyle étant linéaire ou ramifiée et saturée ou non saturée et étant non substituée ou substituée une ou plusieurs fois et p ayant la valeur 0, 1 ou 2, un reste O-aryle ou S(O)_{q}-aryle, la partie aryle étant non substituée ou substituée une ou plusieurs fois et q ayant la valeur 0, 1 ou 2, un reste O-(alkyle en C₁ à C₆)-aryle ou S(O)ᵣ-(alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois et r ayant la valeur 0, 1 ou 2, un groupe CO₂H, C(=O)R¹⁴, un reste alkyle en C₁ à C₁₂, ce reste alkyle étant linéaire ou ramifié et saturé ou non saturé et étant non substitué ou substitué une ou plusieurs fois, un reste CF₃, un reste cycloalkyle en C₃ à C₈, ce reste cycloalkyle étant saturé ou non saturé et étant non substitué ou substitué une ou plusieurs fois, un reste hétérocyclyle, ce reste hétérocyclyle ayant 3, 4, 5, 6 ou 7 chaînons et étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, ou bien R⁶ et R⁷ forment ensemble un groupe Q, Q étant un groupe γ'-CR¹⁵=CR¹⁶-CR¹⁷=CR¹⁸-δ', l'extrémité de Q désignée par γ' étant liée à l'atome γ de carbone du composé de formule générale I et l'extrémité de Q désignée par δ' étant liée à l'atome δ de carbone du composé de formule générale I, et Y et Z sont tels que définis ci-dessus, ou bien R⁶ et R⁹ forment ensemble un groupe T, ce groupe T étant un groupe γ'-CR¹⁹=CR²⁰-CR²¹=CR²²-ε' ou γ'-N=CR²⁰-CR²¹=N-ε', l'extrémité de T désignée par γ' est liée à l'atome γ de carbone du composé de formule générale I et l'extrémité de T désignée par ε' est liée à l'atome ε de carbone du composé de formule générale I et R⁷ et R⁸ sont tels que définis ci-dessus,
R¹⁰ est un reste alkyle en C₁ à C₈, ce reste alkyle étant linéaire ou ramifié et saturé ou non saturé et étant non substitué ou substitué une ou plusieurs fois, un reste cycloalkyle en C₃ à C₈, ce reste cycloalkyle étant saturé ou non saturé et non substitué ou substitué une ou plusieurs fois, un reste hétérocyclyle, ce reste hétérocyclyle ayant 3, 4, 5, 6 ou 7 chaînons et étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois, ou un reste (alkyle en C₁ à C₆)-hétéroaryle, la partie alkyle en C₁ à C₆ étant linéaire ou ramifiée et saturée ou non saturée et étant non substituée ou substituée une ou plusieurs fois et la partie hétéroaryle étant non substituée ou substituée une ou plusieurs fois,
R¹¹ est un groupe NH₂, NH-(alkyle en C₁ à C₆), N-(alkyle en C₁ à C₆)₂, NH-((alkyle en C₁ à C₆)-aryle), N- ( (alkyle en C₁ à C₆)-aryle)₂, NH-aryle, N-(aryle)₂, alkyle en C₁ à C₈, la partie alkyle étant linéaire ou ramifiée et saturée ou non saturée et étant non substituée ou substituée une ou plusieurs fois, un reste cycloalkyle en C₃ à C₈, ce reste cycloalkyle étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois, ou un reste (alkyle en C₁ à C₆)-hétéroaryle, la partie alkyle en C₁ à C₆ étant linéaire ou ramifiée et saturée ou non saturée et étant non substituée ou substituée une ou plusieurs fois et la partie hétéroaryle étant non substituée ou substituée une ou plusieurs fois,
R¹² est un reste alkyle en C₁ à C₈, ce reste alkyle étant linéaire ou ramifié et saturé ou non saturé et étant non substitué ou substitué une ou plusieurs fois, un reste cycloalkyle en C₃ à C₈, ce reste cycloalkyle étant saturé ou non saturé et non substitué ou substitué une ou plusieurs fois, un reste (alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois,
R¹³ est un groupe C(=O)CH₃, C(=O)phényle, C(=O)O-tertiobutyle (t-BOC),
R¹⁴ représente H, un groupe NH₂, NH-(alkyle en C₁ à C₆), N-(alkyle en C₁ à C₆)₂, NH-((alkyle en C₁ à C₆)-aryle), N-((alkyle en C₁ à C₆)-aryle)₂, NH-aryle, N-(aryle)₂, alkyle en C₁ à C₈, la partie alkyle étant linéaire ou ramifiée et saturée ou non saturée et étant non substituée ou substituée une ou plusieurs fois, un reste cycloalkyle en C₃ à C₈, ce reste cycloalkyle étant saturé ou non saturé et non substitué ou substitué une ou plusieurs fois, un reste aryle, ce reste aryle étant non substitué ou substitué une ou plusieurs fois, ou bien un reste (alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois, un reste O-(alkyle en C₁ à C₈), la partie alkyle étant linéaire ou ramifiée et saturée ou non saturée et étant non substituée ou substituée une ou plusieurs fois, un reste O-(cycloalkyle en C₃ à C₈), la partie cycloalkyle étant saturée ou non saturée et non substituée ou substituée une ou plusieurs fois, un reste O-aryle, la partie aryle étant non substituée ou substituée une ou plusieurs fois, ou un reste O-(alkyle en C₁ à C₆)-aryle, la partie alkyle en C₁ à C₆ étant linéaire ou ramifiée et saturée ou non saturée et étant non substituée ou substituée une ou plusieurs fois et la partie aryle étant non substituée ou substituée une ou plusieurs fois,
R¹⁵, R¹⁶, R¹⁷ et R¹⁸ représentent indépendamment les uns des autres H, F, Cl, Br, I, OH, CN, alkyle en C₁ à C₈, le reste alkyle étant linéaire ou ramifié et saturé ou non saturé et étant non substitué ou substitué une ou plusieurs fois, un groupe CO₂H, et
R¹⁹, R²⁰, R²¹ et R²² représentent indépendamment les uns des autres H, F, Cl, Br, I, CN, OH, un reste alkyle en C₁ à C₈, ce reste alkyle étant linéaire ou ramifié et saturé ou non saturé et étant non substitué ou substitué une ou plusieurs fois, ou un groupe CO₂H, ainsi que leurs sels acceptables du point de vue pharmaceutique.

2. Composés de formule générale I suivant la revendication 1 sous forme de leurs racémates, sous forme des énantiomères purs ou sous forme de mélanges des énantiomères ou des diastéréo-isomères dans un rapport de mélange quelconque, ainsi que leurs sels acceptables du point de vue pharmaceutique.

3. Composés de formule générale I suivant l'une des revendications 1 et 2 ou leurs sels acceptables du point de vue pharmaceutique, formule dans laquelle Y représente N et Z représente CR⁹, l'un des restes R¹ et R² représente un groupe OR¹⁰, SR¹⁰, un reste alkyle en C₁ à C₆ ou aryle, l'un des restes R³ et R⁴ représente H ou un reste alkyle en C₁ à C₆, ou bien l'un des restes R¹ et R² forme conjointement avec l'un des restes R³ et R⁴ un groupe W, le groupe W ayant la formule
α'-CH=CH-CH₂-CH₂-β' ; 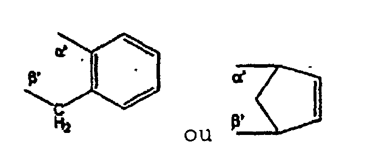 et les deux autres restes de R¹, R², R³ et R⁴ représentent H ou un reste alkyle en C₁ à C₁₂, R⁵ est un reste cycloalkyle en C₃ à C₇, hétéroaryle, C(=O)R¹¹, CO₂H ou CO₂R¹², R⁶ représente H, F, Cl, Br, CN, NO₂, un groupe C(=O)R¹⁴, un reste alkyle en C₁ à C₆, CF₃ ou aryle, R⁷ représente H, F, Cl, Br, CN, NH₂, OH ou un reste alkyle en C₁ à C₆, R⁹ représente H, OH, CF₃ ou un reste alkyle en C₁ à C₆, ou bien R⁶ et R⁹ forment ensemble un groupe T, le groupe T ayant la formule γ'-N=CR²⁰-CR²¹=N-ε', R¹⁰ est un reste alkyle en C₁ à C₈ ou aryle, R¹¹ est un reste aryle, R¹² est un reste alkyle en C₁ à C₆, R¹⁴ est un reste O-(alkyle en C₁ à C₆), R²⁰ représente H ou CO₂H et R²¹ représente H.

4. Composés de formule générale I suivant la revendication 3 ou leurs sels acceptables du point de vue pharmaceutique, l'un des restes R¹ et R² étant un reste O-(CH₂)₂-OH, S-phényle, phényle, 3-méthylphényle, 2,4-diméthylphényle, 4-hydroxyphényle, 4-méthoxyphényle, 4-hydroxy-3-méthoxyphényle, 3,4-diméthoxyphényle ou 2-naphtyle, l'un des restes R³ et R⁴ représente H ou un reste méthyle, ou bien l'un des restes R¹ et R² forme conjointement avec l'un des restes R³ et R⁴ un groupe W, le groupe W ayant pour formule
α'-CH=CH-CH₂-CH₂-β' 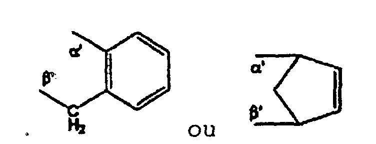 et les deux autres restes de R¹, R², R³ et R⁴ représentent H, R⁵ est un reste cyclopropyle, 2-(C(=O)O-éthyl)-cyclopropyle, cyclohexyle, 2-pyridinyle, C(=O)phényle, CO₂H ou CO₂éthyle, R⁶ représente H, Br, un reste CO₂-éthyle ou méthyle, R⁷ représente H, NH₂, OH ou un reste méthyle et R⁹ représente H, Cl, OH ou CF₃, ou bien R⁶ et R⁹ forment ensemble un groupe T, le groupe T ayant la formule γ'-N=C(CO₂H)-CH=N-ε'.

5. Composés de formule générale I suivant l'une des revendications 3 et 4 ou leurs sels acceptables du point de vue pharmaceutique, ces composés étant choisis parmi les suivants :
ester d'éthylène d'acide 7-bromo-4-(4-méthoxyphényl)-3,4-dihydro-2H-pyrimido[1,2-a]pyrimidine-2-carboxylique,
acide 7-bromo-4-(3,4-diméthoxyphényl)-3,4-dihydro-2H-pyrimido[1,2-a]pyrimidine-2-carboxylique,
acide 7-bromo-4-(4-méthoxyphényl)-3,4-dihydro-2H-pyrimido-[1,2-a]pyrimidine-2-carboxylique,
ester éthylique d'acide 7-bromo-4-(4-hydroxyphényl)-3-méthyl-3,4-dihydro-2H-pyrimido[1,2-a]pyrimidine-2-carboxylique,
ester éthylique d'acide 2-bromo-6,6a,7,11b-tétrahydro-4,5,11c-triazabenzo[c]fluorène-6-carboxylique,
acide 7-bromo-4-(4-méthoxyphényl)-3-méthyl-3,4-dihydro-2H-pyrimido-[1,2-a]pyrimidine-2-carboxylique,
acide 7-bromo-4-(3,4-diméthoxyphényl)-3,4-dihydro-2H-pyrimido-[1,2-a]pyrimidine-2-carboxylique,
acide 7-bromo-4-(4-méthoxyphényl)-3,4-dihydro-2H-pyrimido-[1,2-a]pyrimidine-2-carboxylique,
[7-bromo-4-(2-hydroxyéthoxy)-3,4-dihydro-2H-pyrimido-[1,2-a]pyrimidine-2-yl]-phénylméthanone,
[7-bromo-4-(4-hydroxy-3-méthoxyphényl)-3-méthyl-3,4-dihydro-2H-pyrimido[1,2-a]pyrimidine-2-yl]-phénylméthanone,
[7-bromo-4-(4-méthoxyphényl)-3-méthyl-3,4-dihydro-2H-pyrimido[1,2-a]pyrimidine-2-yl]-phénylméthanone,
[7-bromo-4-(4-hydroxyphényl)-3-méthyl-3,4-dihydro-2H-pyrimido[1,2-a]pyrimidine-2-yl]-phénylméthanone,
[7-bromo-4-(3,4-diméthoxyphényl)-3,4-dihydro-2H-pyrimido-[1,2-a]pyrimidine-2-yl]-phénylméthanone,
[7-bromo-4-(4-méthoxyphényl)-3,4-dihydro-2H-pyrimido-[1,2-a]pyrimidine-2-yl]-phénylméthanone,
2-(7-bromo-2-cyclopropyl-3,4-dihydro-2H-pyrimido[1,2-a]-pyrimidine-4-yloxy)-éthanol,
2-(7-bromo-2-cyclohexyl-3,4-dihydro-2H-pyrimido[1,2-a]-pyrimidine-4-yloxy)-éthanol,
4-(7-bromo-2-cyclohexyl-3-méthyl-3,4-dihydro-2H-pyrimido-[1,2-a]pyrimidine-4-yl)-phénol,
ester éthylique d'acide 7-bromo-4-naphtalène-2-yl-3,4-dihydro-2H-pyrimido[1,2-a]pyrimidine-2-carboxylique,
ester éthylique d'acide 7-bromo-4-m-tolyl-3,4-dihydro-2H-pyrimido[1,2-a]pyrimidine-2-carboxylique,
ester éthylique d'acide 2-[7-bromo-4-(2,4-diméthylphényl)-3,4-dihydro-2H-pyrimido[1,2-a]pyrimidine-2-yl]-cyclopropanecarboxylique,
ester éthylique d'acide 2-[7-bromo-4-(4-méthoxyphényl)-3,4-dihydro-2H-pyrimido[1,2-a]pyrimidine-2-yl]-cyclopropanecarboxylique,
7-bromo-4-phénylsulfanyl-2-pyridine-2-yl-3,4-dihydro-2H-pyrimido[1,2-a]pyrimidine,
2-chloro-6-phénylsulfanyl-8-pyridine-2-yl-7,8-dihydro-6H-pyrimido[1,2-a]pyrmidine-4-ylamine,
6-phénylsulfanyl-8-pyridine-2-yl-7,8-dihydro-6H-pyrimido-[1,2-a]pyrimidine-2,4-diol,
ester éthylique d'acide 6-phénylsulfanyl-8-pyridine-2-yl-2-trifluorométhyl-7,8-dihydro-6H-pyrimido[1,2-a]pyrimidine-3-carboxylique,
3-méthyl-5,8-méthano-9-pyridine-2-yl-5,8,8a,9-tétrahydro-4bH-1,4a,10-triazaphénanthrène-2,4-diol,
5,8-méthano-9-pyridine-2-yl-5,8,8a,9-tétrahydro-4bH-1,4a,10-triazaphénanthrène-2,4-diol,
acide 12-hydroxyl-1,4-méthano-5-pyridine-2-yl-1,4a,5,12b-tétrahydro-4H-6,7,8,11,12a-pentaazabenzo[a]anthracène-10-carboxylique,
3-bromo-9-pyridine-2-yl-7,8,8a,9-tétrahydro-4bH-1,4a,10-triazaphénanthrène.

6. Composés de formule générale I suivant l'une des revendications 1 et 2 ou leurs sels acceptables du point de vue pharmaceutique, Y représentant un groupe CR⁸ et Z représentant N, l'un des restes R¹ et R² représente un groupe OR¹⁰, SR¹⁰, un reste alkyle en C₁ à C₆ ou un reste aryle, R³ et R⁴ représentent H ou un reste alkyle en C₁ à C₆, ou bien l'un des restes R¹ et R² forme un groupe W conjointement avec l'un des restes R³ et R⁴, le groupe W ayant la formule 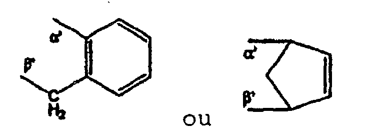 et les deux autres restes de R¹, R², R³ et R⁴ représentent H ou un reste alkyle en C₁ à C₁₂, R⁵ est un reste cycloalkyle en C₃ à C₇, hétéroaryle, C(=O)R¹¹, CO₂H ou CO₂R¹², R⁶ représente H, F, Cl, Br, CN, NO₂, C(=O)R¹⁴, un reste alkyle en C₁ à C₆, CF₃ ou aryle, R⁷ représente H, F, Cl, Br, CN, NH₂, OH ou un reste alkyle en C₁ à C₆, R⁸ représente H, F, Cl, Br, CN, NO₂, un reste O-(alkyle en C₁ à C₆)-aryle, CO₂H, CONH₂ ou alkyle en C₁ à C₆, R¹⁰ est un reste alkyle en C₁ à C₈ ou aryle, R¹¹ est un reste aryle, R¹² est un reste alkyle en C₁ à C₆ et R¹⁴ représente un reste O-(alkyle en C₁ à C₆).

7. Composés de formule générale I suivant la revendication 6 ou leurs sels acceptables du point de vue pharmaceutique, l'un des restes R¹ et R² étant un reste O-(CH₂)₂-OH, S-phényle, méthyle, phényle, 2,4-diméthylphényle, 4-hydroxyphényle, 4-méthoxyphényle, 4-hydroxy-3-méthoxyphényle ou 3,4-diméthoxyphényle, l'un des restes R³ et R⁴ représente H ou un reste méthyle, ou bien l'un des restes R¹ et R² forme un groupe W conjointement avec l'un des restes R³ et R⁴, le groupe W ayant la formule 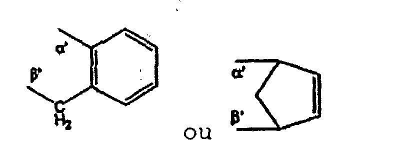 H, R⁵ est un reste cyclopropyle, 2-(C(=O)O-éthyl)-cyclopropyle, cyclohexyle, 2-pyridinyle, 5-méthylfuranne-2-yle, 4-nitrofuranne-2-yle, C(=O)phényle, CO₂H ou CO₂éthyle, R⁶ représente H, Cl, CN ou un reste phényle, R⁷ représente H, NH₂ ou CN et R⁸ représente H, Cl, CN, CO₂H ou CONH₂, ainsi que leurs sels acceptables du point de vue pharmaceutique.

8. Composés de formule générale I suivant l'une des revendications 6 et 7 ou leurs sels acceptables du point de vue pharmaceutique, ces composés étant choisis parmi les suivants :
4-(4-méthoxyphényl)-3-méthyl-2-(5-nitrofuranne-2-yl)-3,4-dihydro-2H-pyrazino[1,2-a]pyrimidine,
ester éthylique d'acide 2-[4-(4-méthoxyphényl)-3-méthyl-3,4-dihydro-2H-pyrazino[1,2-a]pyrimidine-2-yl]-cyclopropanecarboxylique,
4-(4-méthoxyphényl)-3-méthyl-2-(5-méthylfuranne-2-yl)-3,4-dihydro-2H-pyrazino[1,2-a]-pyrimidine,
ester éthylique d'acide 4-(3,4-diméthoxyphényl)-3,4-dihydro-2H-pyrazino[1,2-a]-pyrimidine-2-carboxylique,
ester éthylique d'acide 4-(4-méthoxyphényl)-3,4-dihydro-2H-pyrazino[1,2-a]-pyrimidine-2-carboxylique,
acide 4-(3,4-diméthoxyphényl)-3,4-dihydro-2H-pyrazino-[1,2-a]pyrimidine-2-carboxylique,
acide 4-(4-méthoxyphényl)-3,4-dihydro-2H-pyrazino-[1,2-a]-pyrimidine-2-carboxylique,
4-(4-méthoxyphényl)-2-(5-nitrofuranne-2-yl)-3,4-dihydro-2H-pyrazino[1,2-a]pyrimidine,
ester éthylique d'acide 2-[4-(2,4-diméthylphényl)-3,4-dihydro-2H-pyrazino[1,2-a]pyrimidine-2-yl]-cyclopropane-carboxylique,
ester éthylique d'acide 2-[4-(4-méthoxyphényl)-3,4-dihydro-2H-pyrazino[1,2-a]pyrimidine-2-yl]-cyclopropane-carboxylique,
ester éthylique d'acide 4-(4-hydroxyphényl)-3-méthyl-3,4-dihydro-2H-pyrazino[1,2-a]pyrimidine-2-carboxylique,
ester éthylique d'acide 6,6a,7,11b-tétrahydro-3,5,11c-triazabenzo[c]fluorène-6-carboxylique,
ester éthylique d'acide 4-phénylsulfanyl-3,4-dihydro-2H-pyrazino[1,2-a]pyrimidine-2-carboxylique,
acide 4-phénylsulfanyl-3,4-dihydro-2H-pyrazino[1,2-a]-pyrimidine-2-carboxylique,
acide 4-(4-méthoxyphényl)-3-méthyl-3,4-dihydro-2H-pyrazino-[2,2-a]pyrimidine-2-carboxylique,
acide 4-(3,4-diméthoxyphényl)-3,4-dihydro-2H-pyrazino-[1,2-a]pyrimidine-2-carboxylique,
acide 4-(4-méthoxyphényl)-3,4-dihydro-2H-pyrazino[1,2-a]-pyrimidine-2-carboxylique,
[4-(2-hydroxyéthoxy)-3,4-dihydro-2H-pyrazino[1,2-a]-pyrimidine-2-yl]-phénylméthanone,
[4-(4-hydroxy-3-méthoxyphényl)-3-méthyl-3,4-dihydro-2H-pyrazino[1,2-a]pyrimidine-2-yl]-phénylméthanone,
[4-(4-méthoxyphényl)-3-méthyl-3,4-dihydro-2H-pyrazino-[1,2-a]pyrimidine-2-yl]-phénylméthanone,
[4-(4-hydroxyphényl)-3-méthyl-3,4-dihydro-2H-pyrazino-[1,2-a]-pyrimidine-2-yl]-phénylméthanone,
2-cyclopropyl-4-(3,4-diméthoxyphényl)-3,4-dihydro-2H-pyrazino[1,2-a]pyrimidine,
2-cyclopropyl-4-(4-méthoxyphényl)-3,4-dihydro-2H-pyrazino-[1,2-a]pyrimidine,
2-(2-cyclohexyl-3,4-dihydro-2H-pyrazino[1,2-a]pyrimidine-4-yloxy)-éthanol,
2-cyclohexyl-4-(4-méthoxyphényl)-3,4-dihydro-2H-pyrazino-[1,2-a]pyrimidine,
4-méthyl-2-(5-nitrofuranne-2-yl)-4-phényl-3,4-dihydro-2H-pyrazino[1,2-a]pyrimidine,
ester éthylique d'acide 2-(4-méthyl-4-phényl-3,4-dihydro-2H-pyrazino[1,2-a]pyrimidine-2-yl)-cyclopropanecarboxylique,
4-phényl-2-pyridine-2-yl-3,4-dihydro-2H-pyrazino[1,2-a]-pyrimidine,
acide 4-phényl-2-pyridine-2-yl-3,4-dihydro-2H-pyrazino-[1,2-a]pyrimidine-9-carboxylique,
6-amino-7-chloro-4-phényl-2-pyridine-2-yl-3,4-dihydro-2H-pyrazino[1,2-a]pyrimidine-9-carboxamide,
2-phénylsulfanyl-3-pyridine-2-yl-2,3-dihydro-1H-pyrimido-[1,2-a]quinoléine-10-ol,
acide 4-phénylsulfanyl-2-pyridine-2-yl-3,4-dihydro-2H-pyrazino[1,2-a]pyrimidine-9-carboxylique,
6-amino-7-chloro-4-phénylsulfanyl-2-pyridine-2-yl-3,4-dihydro-2H-pyrazino[1,2-a]pyrimidine-9-carboxamide,
7-phényl-4-phénylsulfanyl-2-pyridine-2-yl-3,4-dihydro-2H-pyrazino[1,2-a]pyrimidine-9-carbonitrile,
5,8-méthano-9-pyridine-2-yl-5,8,8a,9-tétrahydro-4bH-2,4a,10-triazaphénanthrène,
1-chloro-5,8-méthano-9-pyridine-2-yl-5,8,8a,9-tétrahydro-4bH-2,4a,10-triazaphénanthrène-3,4-dicarbonitrile,
ainsi que leurs sels acceptables du point de vue pharmaceutique.

9. Procédé de production de composés de formule générale I suivant l'une des revendications 1 à 8 et de leurs sels acceptables du point de vue pharmaceutique 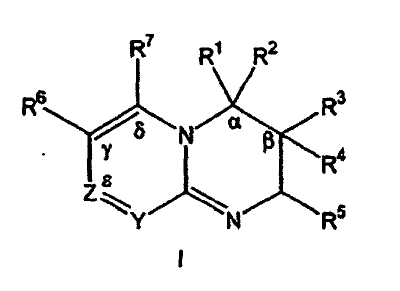 formule dans laquelle Y, Z et R¹ à R⁷ sont tels que définis dans la revendication 1,
**caractérisé en ce qu'**on fait réagir une hétéroarylamine de formule générale II 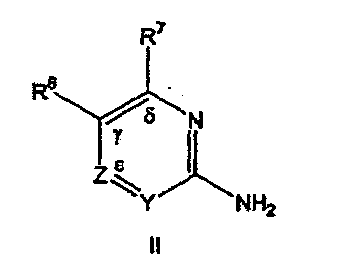 dans laquelle Y, Z, R⁶ et R⁷ sont tels que définis ci-dessus, sous réserve que lorsque R⁶ et R⁷ forment le groupe Q tel que défini ci-dessus, l'extrémité de Q désignée par γ' soit liée à l'atome γ de carbone de l'hétéroarylamine de formule générale II et que l'extrémité de Q désignée par δ' soit liée avec l'atome δ de carbone de l'hétéroarylamine de formule générale II, et que lorsque R⁶ et R⁹ forment le groupe T tel que défini ci-dessus, l'extrémité désignée par γ' soit liée avec l'atome γ de carbone de l'hétéroarylamine de formule générale II et l'extrémité désignée par ε' soit liée avec l'atome ε de carbone de l'hétéroarylamine de formule générale II,
avec un aldéhyde de formule générale III 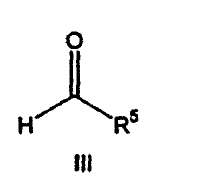 dans laquelle R⁵ est tel que défini ci-dessus,
et une oléfine de formule générale IV 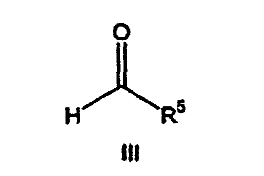 dans laquelle R¹, R², R³ et R⁴ sont tels que définis ci-dessus, sous réserve que lorsque l'un des restes R¹ et R² forme un groupe W conjointement avec l'un des restes R³ et R⁴, l'extrémité désignée par α' de W soit liée avec l'atome α de carbone de l'oléfine de formule générale IV et l'extrémité désignée par β' du groupe W soit liée avec l'atome β de carbone de l'oléfine de formule générale IV, en présence d'un acide.

10. Procédé suivant la revendication 9, **caractérisé en ce que** la réaction de l'hétéroarylamine de formule générale II avec l'aldéhyde de formule générale III et l'oléfine de formule générale IV est conduite selon un procédé en récipient unique.

11. Procédé suivant l'une des revendications 9 et 10, **caractérisé en ce que** l'acide est l'acide trifluoracétique.

12. Procédé suivant l'une des revendications 9 à 11, **caractérisé en ce que** la réaction est conduite dans un solvant organique à une température de 0 à 100°C et pendant une durée de réaction de 0,25 à 12 heures.

13. Procédé suivant l'une des revendications 9 à 12, **caractérisé en ce que** la réaction est conduite à une température de 15 à 40°C.

14. Procédé suivant l'une des revendications 9 à 13, **caractérisé en ce que** le solvant organique est l'acétonitrile.

15. Bibliothèque de substances, contenant au moins un composé de formule générale I suivant l'une des revendications 1 à 8.

16. Médicament, contenant au moins un composé de formule générale I ou l'un de ses sels acceptables du point de vue pharmaceutique suivant l'une des revendications 1 à 8.

17. Utilisation d'un composé de formule générale I suivant l'une des revendications 1 à 8 ou de l'un de ses sels acceptables du point de vue pharmaceutique pour la préparation d'un médicament destiné au traitement de la douleur.

18. Utilisation d'un composé de formule générale I suivant l'une des revendications 1 à 8 ou de l'un de ses sels acceptables du point de vue pharmaceutique pour la préparation d'un médicament destiné au traitement de l'incontinence urinaire, du prurit, des acouphènes et/ou de la diarrhée.

19. Composition pharmaceutique, qui contient au moins un composé de formule générale I ou l'un de ses sels acceptables du point de vue pharmaceutique suivant l'une des revendications 1 à 8 ainsi qu'au moins une substance auxiliaire pharmaceutique.
